# DEMANDE DE BREVET EUROPEEN

(11) **EP 3 763 355 A1**
(43) Date de publication de la demande: **13.01.2021**
(21) Numéro de dépôt: 20185331.4
(22) Date de dépôt: 10.07.2020
(51) Int. Cl.: A61K 8/9789, A61Q 19/06, A61K 8/368, A61K 8/64, A61K 36/185, A61K 36/54, A61K 36/85, A61P 17/02

(54) **COMPOSITION COMPRENANT DES POLYPHÉNOLS DE GRAINES DE PASSIFLORE, DES PEPTIDES D'AVOCAT ET UN EXTRAIT D'HAMAMÉLIS ET UTILISATION POUR TRAITER ET/OU PRÉVENIR LES VERGETURES**

(30) Priorité: 12.07.2019 FR 1907889
(71) Demandeur: Laboratoires Expanscience, 92048 Paris La Défense Cedex (FR)
(72) Inventeur: BREDIF, Stéphanie, 28210 CROISILLES (FR); BELLEMERE, Gaëlle, 28300 CHAMPHOL (FR)
(74) Mandataire: Regimbeau

(57) **Abrégé**

La présente invention concerne des compositions, notamment cosmétiques, comprenant un extrait de polyphénols de graines de passiflore, un extrait de peptides d'avocat et un extrait d'hamamélis. La présente invention porte également sur l'utilisation cosmétique et/ou esthétique d'une telle composition et sur une méthode de soin cosmétique et/ou esthétique utilisant une telle composition, pour prévenir l'apparition des vergetures sur la peau et/ou réduire les vergetures sur la peau. L'invention concerne en outre une composition pharmaceutique comprenant de tels extraits, notamment pour son utilisation dans la prévention ou le traitement des vergetures de la peau.

## Description

### DOMAINE DE L'INVENTION

La présente invention se situe dans le domaine de la cosmétique et de la dermatologie. Elle concerne des compositions, notamment cosmétiques, comprenant un extrait de polyphénols de graines de passiflore, un extrait de peptides d'avocat et un extrait d'hamamélis. La présente invention porte également sur l'utilisation cosmétique et/ou esthétique d'une telle composition et sur une méthode de soin cosmétique et/ou esthétique utilisant une telle composition, pour prévenir l'apparition des vergetures sur la peau et/ou réduire les vergetures sur la peau. L'invention concerne en outre une composition pharmaceutique comprenant de tels extraits, notamment pour son utilisation dans la prévention ou le traitement des vergetures de la peau.

### ETAT DE LA TECHNIQUE

Les vergetures (ou *striae distensa*) se présentent sous la forme de stries linéaires ou fusiformes, pouvant présenter une couleur variant du rouge vif au pourpre ("*striae rubrae"* ou vergetures immatures), qui peuvent apparaître suite à une distension exagérée de la peau et/ou à une modification hormonale (par exemple gain de poids, grossesse, adolescence).Elles sont pratiquement toujours multiples et très souvent symétriques, avec une surface pouvant présenter un aspect boursouflé, reflétant un état inflammatoire. Elles tendent à changer de couleur au cours du temps et à prendre un aspect blanc nacré (donnant le nom de *"striae alba"*) et la forme de dépressions cutanées visibles et palpables, d'aspect très souvent fripé (vergetures matures).

Les vergetures concernent le plus souvent les femmes, pendant la puberté ou lors d'une grossesse. Ainsi, 50% à 80% de femmes enceintes environ vont développer des vergetures. Les vergetures liées à la grossesse siègent surtout sur la face antérieure de l'abdomen, mais existent aussi au niveau des seins, des cuisses et des hanches. Les vergetures peuvent également apparaître lors d'états physiologiques ou pathologiques tels que l'obésité, un régime alimentaire relativement intensif, la tuberculose et la fièvre typhoïde. Toutefois, si les vergetures peuvent être des symptômes de pathologies, elles ne constituent pas en elles-mêmes un risque pour la santé, et ne sont la cause d'aucune pathologie. En fait, les gênes les plus importantes et les plus fréquentes sont esthétiques, ce qui peut contraindre certaines personnes à cacher les zones du corps affectées, générant ainsi un inconfort (vêtements longs etc...).

Les principaux facteurs déclenchant l'apparition de vergetures sont l'inflammation, la contrainte mécanique et l'environnement hormonal. Ils provoquent un étirement, une réorientation et une réorganisation des fibres de collagène et d'élastine, sans rupture du tissu de soutien. Ainsi, plusieurs équipes ont montré d'une part une augmentation de la rugosité au niveau de la vergeture avec apparition d'un microrelief d'orientation perpendiculaire à l'axe de la vergeture, et d'autre part une augmentation de l'index d'anisotropie du relief (orientation privilégiée du microrelief). Les données montrent en effet que les faisceaux de collagène sont plus épais et alignés parallèlement à la surface de la peau au niveau de la vergeture (orientés dans le sens de la largeur de la vergeture). Les fibres élastiques (principalement constituées d'élastine et de fibrilline-1) sont plus fines, incurvées et en plus petite quantité. L'épaisseur de l'épiderme n'est pas diminuée mais un aplatissement de la jonction dermo-épidermique (JDE) a été observé, avec une altération des fibres oxytalanes (Gilmore et al., 2012, Math. Biosciences 240: 141-147 ; Rolfe et al., Australas J. Dermatol., 2012 ; Bertin et al., Skin Res. Technol., 2013 ; Lid-Din et al., 2013, Arch Dermatol Res, 305: 603-617).

Il a également été montré que la prolifération des fibroblastes est ralentie dans les vergetures (notamment lors de la grossesse ou de l'adolescence). Une diminution de la synthèse de collagène I et III, d'élastine, de fibrilline-1 et de fibronectine par les fibroblastes a en outre été observée (Mitts et al., 2005 ; Gilmore et al., 2012, Math. Biosciences 240: 141-147 ; Reymermier et al ., SOFW Journal, 2012 ; Al. Himdani et al., BJD, 2014). Ainsi, les fibres élastiques et notamment l'élastine semblent jouer un rôle particulièrement important dans les mécanismes d'apparition des vergetures. Une analyse génomique à grande échelle a ainsi révélé que l'on trouve une région située en amont du gène de l'élastine, ainsi qu'une région en amont du gène de la fibronectine, parmi les régions du génome qui semblent associées à l'apparition des vergetures, chez des sujets sains (Tung et al., JID, 2013).

Le manque d'eau dans les cellules peut également jouer un rôle. En effet, dès les premiers signes de déshydratation, les fibres du collagène forment des ramifications nombreuses et rapprochées qui, à force de tension, perdent de leur élasticité et se cassent de façon visible au niveau du derme.

Les traitements disponibles visent à prévenir la formation des vergetures ainsi qu'à atténuer et améliorer leur aspect (traitements cosmétiques et esthétiques). Les traitements sont essentiellement locaux : traitements topiques avec de la trétinoïne (all-trans acide rétinoïque) ou des acides de fruits, traitements peeling ou encore laser. La trétinoïne aurait une action anti-vergetures tendant à restaurer essentiellement le réseau des fibrillines. Par exemple, la demande WO 00/19974 décrit une méthode cosmétique de prévention de l'apparition et/ou de traitement des vergetures. La composition cosmétique proposée comprend au moins un agent anti-vergetures choisi parmi des peptides de soja, des tripeptides constitués des acides aminés glycine, histidine et lysine, et les mélanges de ces peptides. La demande WO 2010/052327 décrit quant à elle une méthode de prévention et/ou de traitement des vergetures à l'aide d'une composition cosmétique comprenant de l'arabinogalactane.

Cependant, le développement de tels traitements est freiné par l'absence d'outils permettant d'évaluer, *in vitro,* l'effet de ces compositions sur la peau vergeturée de manière fidèle, dans les conditions les plus proches possibles de la vergeture *in vivo.* Or de tels outils permettraient non seulement de tester l'efficacité de compositions anti-vergetures dans des conditions physiologiques, mais également leur innocuité, en amont d'une application sur un sujet.

Il existe donc toujours un réel besoin de nouvelles compositions permettant de prévenir et/ou de traiter efficacement les vergetures, avec une bonne tolérance cutanée et une action inhibitrice des différents facteurs d'apparition des vergetures, prouvées dans des conditions physiologiques.

La présente invention permet de répondre à ce besoin. Les présents Inventeurs ont utilisé un modèle de peau inédit, mimant fidèlement la physiologie de la peau humaine vergeturée. Dans ce modèle de peau, l'orientation des fibres de collagène et l'organisation du réseau des fibres élastiques est modifiée comme dans la vergeture in vivo, avec un raccourcissement des fibres d'élastine et une orientation des fibres d'élastine et de fibres de collagène dans le sens de l'étirement. Les données montrent en outre un aplatissement de la jonction dermo-épidermique (JDE), avec une altération des fibres oxytalanes. De plus, les données montrent que les niveaux d'expression du collagène III sont diminués alors qu'une inflammation est induite, avec une augmentation du niveau d'expression du marqueur spécifique de la vergeture alpha-Smooth Muscle Actin (αSMA), ainsi que du relargage des cytokines de l'inflammation IL6, IL8 et IL1α, et de la métalloprotéinase MMP1.

Les Inventeurs ont ainsi montré que, de manière surprenante, l'application d'une composition comprenant un extrait de polyphénols de graines de passiflore, un extrait de peptides d'avocat et un extrait d'hamamélis permet non seulement de restaurer l'organisation et l'orientation des fibres de collagène, des fibres d'élastine et des fibres oxytalanes, mais également de restaurer les niveaux d'expression du marqueur spécifique de la vergeture aSMA, du collagène III, des cytokines de l'inflammation IL6, IL8 et IL1α, et de la métalloprotéinase MMP1. Une étude menée par les Inventeurs chez des femmes enceintes et après accouchement a permis de confirmer ces données. En effet, cette étude montre qu'une telle composition permet de prévenir la formation des vergetures et de réduire les vergetures de manière efficace, tout en étant très bien tolérée. Cette composition améliore de manière significatives les aspects inesthétiques (visuels, tactiles) des vergetures existantes ainsi que les gênes associées, et limite l'apparition des aspects inesthétiques et incommodant des vergetures. Ces résultats sont d'autant plus inattendus que l'effet anti-vergetures de chacun des actifs utilisés, l'extrait de polyphénols de graines de passiflore, l'extrait de peptides d'avocat et l'extrait d'hamamélis, n'était pas connu. Les données montrent également qu'une telle composition a effet hydratant significatif, renforçant ainsi son action anti-vergetures. Les données obtenues à partir d'un modèle de peau reproduisant de façon fidèle la physiologie de la vergeture montrent en outre que la combinaison de ces trois actifs naturels a un effet anti-vergetures supérieur à celui de chacun de ces actifs seuls. En particulier, l'application de la combinaison d'un extrait de polyphénols de graines de passiflore, d'un extrait de peptides d'avocat avec un extrait d'hamamélis conduit à une restauration des niveaux d'expression des marqueurs de l'inflammation et du marqueur spécifique de la vergeture alpha-Smooth Muscle Actin (αSMA) significativement plus proche de la peau normale non étirée.

### RESUME DE L'INVENTION

Dans le cadre de la présente invention, les Inventeurs ont mis en évidence, de manière tout à fait surprenante, qu'une composition comprenant un extrait de polyphénols de graines de passiflore, un extrait de peptides d'avocat et un extrait d'hamamélis permet de prévenir la formation de vergetures et de réduire les vergetures existantes sur la peau, notamment chez des femmes enceintes et post-partum, avec une grande efficacité. Ces résultats sont d'autant plus inattendus que les extraits de polyphénols de graines de passiflore, les extraits de peptides d'avocat et les extraits d'hamamélis, n'étaient pas connus pour avoir un effet anti-vergetures.

La présente invention concerne donc une nouvelle composition comprenant un extrait de polyphénols de graines de passiflore, un extrait de peptides d'avocat et un extrait d'hamamélis.

La présente invention concerne également l'utilisation cosmétique et/ou esthétique d'une telle composition, ainsi qu'une méthode de soin cosmétique et/ou esthétique utilisant une telle composition, pour prévenir l'apparition des vergetures sur la peau et/ou réduire les vergetures sur la peau.

L'invention concerne en outre une composition pharmaceutique comprenant de tels extraits, notamment pour son utilisation dans la prévention ou le traitement des vergetures de la peau.

### DESCRIPTION DETAILLEE DE L'INVENTION

### Définitions

Par « vergeture" ou par « *striae distensa »,* on entend une atrophie cutanée de formes diverses (saillante, plane ou déprimée), molle et dépressible. Préférentiellement, les vergetures se présentent sous forme des stries linéaires ou fusiformes. Lesdites stries peuvent apparaître suite à une distension exagérée de la peau et/ou à une modification hormonale (par exemple gain de poids, grossesse, adolescence). Elles peuvent éventuellement apparaître aussi lors d'états physiologiques ou pathologiques tels que l'obésité, la tuberculose, la fièvre typhoïde, le syndrome de Prader-Willi, le syndrome de Laurence-Moon Biedel, le syndrome d'Ehlers-Danlos, le syndrome de Cushing, le syndrome de Marfan, le syndrome de Beals (ou syndrome CCA (Contractures - arachnodactylie congénital)). Leur longueur peut varier de moins de 1 cm à plusieurs centimètres et leur largeur de moins d'un 1 mm à environ 2,5 cm. Sont inclues dans les vergetures telles qu'on les entend ici les vergetures récentes (également dites immatures ou *"striae rubrae*") dont la couleur varie souvent du rouge vif au pourpre (mais certaines ne seront jamais colorées), dont la surface peut présenter un aspect boursouflé. Sont également inclues les vergetures matures (ou *"striae alba*"), ayant pris une teinte blanc nacré et la forme de dépressions cutanées visibles et palpables, d'aspect très souvent fripé. Préférentiellement, les vergetures au sens de la présente invention présentent des modifications physiologiques comparativement à la peau normale, de préférence la peau normale d'un sujet appartenant à la même tranche d'âge que la peau vergeturée considérée. Avantageusement, ces modifications physiologiques comprennent une orientation des fibres de collagène et/ou des fibres d'élastine et une organisation du réseau des fibres élastiques modifiée comparativement à la peau normale. Alternativement ou en combinaison, ces modifications physiologiques comprennent un aplatissement de la jonction dermo-épidermique (JDE), et/ou une altération des fibres oxytalanes, en comparaison avec la peau normale. Ces modifications physiologiques peuvent également comprendre une diminution des niveaux d'expression du collagène III et/ou une augmentation de la production des cytokines de l'inflammation IL6, TNF-alpha, IL8 et IL1α, ainsi que de la métalloprotéinase MMP1, et du marqueur spécifique de la vergeture αSMA (alpha-Smooth Muscle Actin), comparativement à la peau normale. A titre d'exemple, les tranches d'âge des sujets peuvent être définies comme suit : les nouveau-nés, dont l'âge est compris entre 0 et 1 mois ; les nourrissons, dont l'âge est supérieur à 1 mois et va jusqu'à 2 ans ; les enfants dont l'âge est supérieur à 2 ans et va jusqu'à 16 ans, les adolescents, dont l'âge est compris entre 12 et 18 ans ; et les adultes, dont l'âge est supérieur à 16 ans.

Par « peau normale » on entend une peau ne présentant pas de vergetures, de préférence chez un sujet sain, avantageusement chez un sujet ayant une peau saine, notamment une peau ne présentant pas de pathologie cutanée.

Par « peau saine », « muqueuse saine » ou « cuir chevelu sain », on entend une zone de peau, de muqueuse ou de cuir chevelu respectivement, dite « non pathologique » par un dermatologue, c'est à dire ne présentant pas d'infection, de maladie ou d'affection cutanée telle que candidose, impétigo, psoriasis, eczéma, acné ou dermatite, ou encore de plaies ou de blessures.

Par « réduction des vergetures (sur la peau) » ou « diminution des vergetures » ou « régression des vergetures » ou encore « atténuation des vergetures » on entend toute amélioration de l'état physiologique de la peau ou de son apparence, notamment au niveau des vergetures. On entend en particulier au moins une amélioration/réduction/régression/atténuation d'au moins une des caractéristiques des vergetures telles que définies ci-dessus, comparativement à la peau normale telle que définie ci-dessus (avantageusement la peau du sujet considéré, située au niveau des zones non vergeturées). Avantageusement, l'amélioration/réduction/régression/atténuation d'au moins une des caractéristiques des vergetures comprend, consiste essentiellement en, ou consiste en ce qu'au moins l'un des critères choisis parmi la liste suivante est rempli :
- la diminution de la taille (largeur et/ou longueur) des vergetures ;
- la diminution du nombre des vergetures (comprenant notamment la diminution du nombre de stries par unité de surface de peau) ;
- l'atténuation des aspects inesthétiques des vergetures, comprenant notamment au moins l'un des critères choisis parmi :
   - l'atténuation de la couleur des vergetures (par exemple dans laquelle la couleur de la vergeture est modifiée pour se rapprocher de la couleur de la peau au niveau des zones non vergeturées) ;
   - l'amélioration de l'aspect visuel et/ou tactile des vergetures ; et
   - l'atténuation du relief des vergetures (le relief comprenant notamment les dépressions, les boursouflures, la rugosité, la granulosité, le microrelief et l'anisotropie ; de préférence dans laquelle le relief de la vergeture est modifié pour se rapprocher du relief de la peau au niveau des zones non vergeturées, par exemple dans laquelle les dépressions, les boursouflures, la rugosité et/ou la granulosité sont atténuées au niveau des vergetures, l'index d'anisotropie du relief est diminué au niveau des vergetures (diminution de l'orientation privilégiée du microrelief), le microrelief est plus homogène;
- l'atténuation des aspects gênants, incommodants et/ou inconfortables (comprenant par exemple l'atténuation des démangeaisons, des sensations de tiraillement, des sensations d'inconfort) ;
- la diminution de l'étirement et/ou de la distension de la peau, de la perte de fermeté et/ou de la perte de l'élasticité et/ou de la perte de la souplesse de la peau ; et
- la diminution/l'atténuation des modifications physiologiques propres aux vergetures, telles que définis ci-dessus, comprenant notamment au moins l'un des critères de réduction des vergetures choisis parmi :
   - Une amélioration de l'organisation et/ou de l'orientation d'au moins un groupe de fibres choisi parmi les fibres de collagène, les fibres d'élastine et les fibres oxytalanes (de préférence une restauration de l'organisation et/ou de l'orientation d'au moins un groupe de fibres choisi parmi les fibres de collagène, les fibres d'élastine et les fibres oxytalanes) ;
   - une réduction de l'aplatissement de la jonction dermo-épidermique (JDE) (de préférence une restauration de la JDE) ;
   - une réduction de l'altération des fibres élastiques, en particulier des fibres oxytalanes (de préférence une restauration desdites fibres) ;
   - une modification des niveaux d'expression d'au moins un marqueur de l'organisation des fibres matricielles du derme, de préférence choisi parmi le gène codant le collagène I, le gène codant le collagène III, et les marqueurs des fibres élastiques choisis parmi le gène codant pour la fibrilline 1 et le gène codant pour l'élastine (de préférence une augmentation des niveaux d'expression d'au moins un marqueur de l'organisation des fibres matricielles du derme) ;
   - une modification des niveaux d'expression d'au moins l'un des marqueurs de l'inflammation choisis parmi IL6, IL8, IL1α, TNF-alpha et la métalloprotéinase MMP1 (de préférence une diminution des niveaux d'expression d'au moins l'un des marqueurs de l'inflammation choisis parmi IL6, IL8, IL1α, TNF-alpha et MMP1) ;
   - une modification du niveau d'expression du marqueur spécifique de la vergeture αSMA (alpha-Smooth Muscle Actin) ;
   comparativement à la peau normale telle que définie ci-dessus (avantageusement la peau du sujet considéré, située au niveau des zones non vergeturées).

Préférentiellement, au moins l'un de ces critères listés ci-dessus est rempli, préférentiellement encore au moins 2, préférentiellement encore au moins 3, préférentiellement encore au moins 4, préférentiellement encore au moins 5, préférentiellement encore au moins 6, préférentiellement encore au moins 7, préférentiellement encore au moins 8, préférentiellement encore au moins 9, préférentiellement encore au moins 10 des critères listés ci-dessus sont remplis, plus préférentiellement encore chacun des critères listés ci-dessus est rempli.

Les méthodes d'évaluation de chacun des critères listés ci-dessus sont décrites plus bas. Par « prévention des vergetures (sur la peau) » ou « prévention de l'apparition des vergetures » on entend la diminution du risque d'apparaitre, de développer ou d'amplifier des vergetures ou encore l'évitement d'apparaitre, de développer ou d'amplifier des vergetures. Autrement dit, on entend la diminution du risque d'apparaitre, de développer ou d'amplifier les caractéristiques des vergetures telles que définis ci-dessus, comparativement à la peau normale telle que définie ci-dessus (avantageusement la peau du sujet considéré, située au niveau des zones non vergeturées). Avantageusement, la prévention des vergetures comprend, consiste essentiellement en, ou consiste en ce qu'au moins l'un des critères de prévention des vergetures choisis parmi la liste suivante est rempli :
- pas d'augmentation de la taille (largeur et/ou longueur) des vergetures ;
- pas d'augmentation du nombre des vergetures (comprenant notamment le nombre de stries par unité de surface de peau) ;
- pas d'augmentation des aspects inesthétiques des vergetures, comprenant notamment au moins l'un des critères choisis parmi :
   - la couleur des vergetures (par exemple dans laquelle la couleur de la vergeture est maintenue proche de la couleur de la peau au niveau des zones non vergeturées) ;
   - l'aspect visuel et/ou tactile des vergetures ; et
   - le relief des vergetures (le relief comprenant notamment les dépressions, les boursouflures, la rugosité, la granulosité, le microrelief et l'anisotropie ; de préférence dans laquelle le relief de la vergeture est maintenu proche du relief de la peau au niveau des zones non vergeturées, par exemple dans laquelle les dépressions et/ou les boursouflures sont évitées ; la rugosité, la granulosité et/ou l'index d'anisotropie du relief sont maintenus (peu ou pas d'orientation privilégiée du microrelief), l'homogénéité du microrelief est maintenue;
- pas d'augmentation des aspects gênants, incommodants et/ou inconfortables (comprenant par exemple les démangeaisons, les sensations de tiraillement, les sensations d'inconfort) ;
- pas d'augmentation de l'étirement et/ou de la distension de la peau, de la perte (et/ou diminution) de fermeté et/ou de la perte (et/ou diminution) de l'élasticité et/ou de la perte (et/ou diminution) de la souplesse de la peau ; et
- pas d'augmentation des modifications physiologiques propres aux vergetures, telles que définis ci-dessus, comprenant notamment au moins l'un des critères choisis parmi :
   - l'organisation et/ou l'orientation d'au moins un groupe de fibres choisi parmi les fibres de collagène, les fibres d'élastine et les fibres oxytalanes (de préférence l'organisation et/ou l'orientation d'au moins un groupe de fibres choisi parmi les fibres de collagène, les fibres d'élastine et les fibres oxytalanes est maintenue) ;
   - l'épaisseur de la jonction dermo-épidermique (JDE) (de préférence l'épaisseur de la JDE est maintenue) ;
   - l'état des fibres élastiques, en particulier des fibres oxytalanes (de préférence l'état desdites fibres est maintenu) ;
   - les niveaux d'expression d'au moins un marqueur de l'organisation des fibres matricielles du derme, de préférence choisi parmi le gène codant le collagène I, le gène codant le collagène III, et les marqueurs des fibres élastiques choisis parmi le gène codant pour la fibrilline 1 et le gène codant pour l'élastine (de préférence le niveau d'expression d'au moins un marqueur de l'organisation des fibres matricielles du derme est maintenu) ;
   - les niveaux d'expression d'au moins l'un des marqueurs de l'inflammation choisis parmi IL6, IL8, IL1α, TNF-alpha et la métalloprotéinase MMP1 (de préférence les niveaux d'expression d'au moins l'un des marqueurs de l'inflammation choisis parmi IL6, IL8, IL1α, TNF-alpha et MMP1 sont maintenus) ;
   - le niveau d'expression du marqueur spécifique de la vergeture αSMA (de préférence le niveau d'expression de αSMA est maintenu) ;
   comparativement à la peau normale telle que définie ci-dessus (avantageusement la peau du sujet considéré, située au niveau des zones non vergeturées) .

Préférentiellement, au moins l'un de ces critères listés ci-dessus est rempli, préférentiellement encore au moins 2, préférentiellement encore au moins 3, préférentiellement encore au moins 4, préférentiellement encore au moins 5, préférentiellement encore au moins 6, préférentiellement encore au moins 7, préférentiellement encore au moins 8, préférentiellement encore au moins 9, préférentiellement encore au moins 10 des critères listés ci-dessus sont remplis, plus préférentiellement encore chacun des critères listés ci-dessus est rempli.

Les méthodes d'évaluation de chacun des critères listés ci-dessus sont décrites plus bas. Par « effet cosmétique et/ou esthétique des vergetures » ou « effet cosmétique et/ou esthétique indésirable des vergetures », on entend au moins l'un des effets choisis parmi:
- l'augmentation de la taille (largeur et/ou longueur) des vergetures ;
- l'augmentation du nombre des vergetures (comprenant notamment la diminution du nombre de stries par unité de surface de peau) ;
- l'augmentation des aspects inesthétiques des vergetures, comprenant notamment au moins l'un des effets choisis parmi :
   - les modifications de la couleur des vergetures ;
   - les modifications de l'aspect visuel et/ou tactile des vergetures ; et
   - les modifications du relief des vergetures (le relief comprenant notamment les dépressions, les boursouflures, la rugosité, la granulosité, le microrelief et l'anisotropie) ;
- les aspects gênants, incommodants et/ou inconfortables (comprenant par exemple les démangeaisons, les sensations de tiraillement, les sensations d'inconfort) ;
- l'étirement et/ou de la distension de la peau,
- la perte et/ou la diminution de la fermeté et/ou de l'élasticité et/ou de la souplesse de la peau ; et
- les modifications physiologiques propres aux vergetures, tels que définies ci-dessus, comprenant notamment au moins l'un des effets des vergetures choisis parmi :
   - Une modification de l'organisation et/ou de l'orientation d'au moins un groupe de fibres choisi parmi les fibres de collagène, les fibres d'élastine et les fibres oxytalanes ;
   - Une modification de l'organisation de la jonction dermo-épidermique (JDE), en particulier un aplatissement de la JDE ;
   - Une modification des fibres élastiques, en particulier des fibres oxytalanes ;
   - une modification (de préférence une diminution) du niveau d'expression d'au moins un marqueur de l'organisation des fibres matricielles du derme, de préférence choisi parmi le gène codant le collagène I, le gène codant le collagène III, et les marqueurs des fibres élastiques choisis parmi le gène codant pour la fibrilline 1 et le gène codant pour l'élastine ;
   - une modification (de préférence une augmentation) des niveaux d'expression d'au moins l'un des marqueurs de l'inflammation choisis parmi IL6, IL8, IL1α, TNF-alpha et la métalloprotéinase MMP1 ;
- une modification (de préférence une augmentation) du niveau d'expression du marqueur spécifique de la vergeture αSMA (alpha-Smooth Muscle Actin) comparativement à la peau normale telle que définie ci-dessus (avantageusement la peau du sujet considéré, située au niveau des zones non vergeturées).

Les méthodes d'évaluation de chacun des effets listés ci-dessus sont décrites plus bas.

Par « réduction d'un effet cosmétique et/ou esthétique des vergetures » ou « réduction d'un effet cosmétique et/ou esthétique indésirable des vergetures », au moins une amélioration/réduction/régression/atténuation d'au moins un des effets cosmétiques et/ou esthétiques des vergetures tels que définis ci-dessus, comparativement à la peau normale telle que définie ci-dessus (avantageusement la peau du sujet considéré, située au niveau des zones non vergeturées, traitée ou non par un produit anti-vergeture) ; ou encore comparativement à la peau vergeturée du sujet considéré, non traitée par un produit anti-vergetures ; ou encore comparativement à une peau vergeturée de référence.

Par « prévention d'un effet cosmétique et/ou esthétique des vergetures » ou « prévention d'un effet cosmétique et/ou esthétique indésirable des vergetures », on entend l'évitement et/ou la diminution du risque d'apparaitre, de développer ou d'amplifier au moins un des effets cosmétiques et/ou esthétiques des vergetures tels que définis ci-dessus, comparativement à la peau normale telle que définie ci-dessus (avantageusement la peau du sujet considéré, située au niveau des zones non vergeturées, traitée ou non par un produit anti-vergeture) ; ou encore comparativement à la peau vergeturée du sujet considéré, non traitée par un produit anti-vergetures ; ou encore comparativement à une peau vergeturée de référence.

Par « polyphénols » ou « composés polyphénoliques » on entend des composés appartenant à la famille de molécules organiques largement présente dans le règne végétal. Ils sont caractérisés par la présence d'au moins deux groupes phénoliques associés en structures plus ou moins complexes, généralement de haut poids moléculaire. Les groupes phénoliques sont des composés chimiques aromatiques portant une fonction hydroxyle -OH. Les polyphénols sont essentiellement constitués par les produits du métabolisme secondaire des plantes.

Par « graines de passiflore » ou « graines de maracuja » on entend les graines (soit la structure qui contient et protège l'embryon végétal, souvent contenue dans un fruit qui permet sa dissémination) d'une plante appartenant à la famille des passiflores (*Passiflora*). Les plantes de passiflores se présentent sont forme d'arbrisseaux ou herbes grimpantes. Les feuilles sont alternes, parfois simples, lobées ou palmées. Les fleurs peuvent attendre 9 cm de diamètre, sont bisexuelles ou unisexuelles et régulières. Elles sont blanches et violettes et présentent des appendices pétaloïdes fins, garnis d'appendices filiformes. Le fruit de 4 à 5 cm de longueur est ovalaire et souvent de couleur jaune à orangée. Les espèces les plus répandues sont notamment *Passiflora incarnata* (*P. incarnata*) et *Passiflora edulis* (P. edulis). On trouve également les espèces *P. foetida* et *P. suberosa.* Par exemple, les graines constituent 6 à 12% du fruit de *P. Edulis.* Avantageusement, les graines de passiflore contiennent :
- des polyphénols dont le Piceatannol (structure proche du resveratrol) et son dimère la scirpusine B (S. Sano ; K. Sugiyama ; T. Ito, 2011) et des substances à effets vasorelaxant et anti-oxydant ;
- de l'huile, (rendement de 15%-20% après extraction par solvant) contenant des phytostérols (environ 0.2% dont campestérol, stigmastérol, sitostérol, avenasterol) ; 60 à 73% d'acide linoléique (oméga 6), 14% à 20% d'acide oléique et 465 ppm de tocophérols (G. Piobom, N. Barou et al, 2006 ; R. de V.V. Lopes et al.) ;
- des sucres et des protéines.

Par « polyphénols de graines de passiflore » ou « polyphénols de graines de maracuja » ou « polyphénols de maracuja » on entend les polyphénols naturellement présents dans les graines de passiflore.

Par « peptide » on entend un polymère d'acides aminés reliés entre eux par des liaisons peptidiques.

Par « avocat » on entend le fruit de l'avocatier. L'avocatier *(Persea americana)* est une espèce d'arbres fruitiers de la famille des Lauracées. Les espèces d'avocatier incluent notamment *Hass, Fuerte, Ettinger, Bacon, Nabal, Anaheim, Lula, Reed, Zutano, Queen, Criola Selva, Mexicana Canta, Region Dschang, Hall, Booth, Peterson,* et *Collinson Red.* L'avocatier est un arbre de taille moyenne qui peut atteindre vingt mètres de hauteur. Cependant, il mesure en général environ dix mètres. Sa cime est ample et touffue, son tronc est recouvert d'une écorce grisâtre et crevassée. Les feuilles sont alternes, elliptiques, entières, à bords lisses, acuminées elles mesurent 20-30 cm sur 10-15 cm, la base est pointue, la face ventrale est vert foncé, brillant, les nervures sont vert clair, la face dorsale est terne, glauque clair à blanc, les feuilles juvéniles, légèrement violacées, sont duveteuses. Le pétiole fait 3-5 cm. Elles tombent tous les ans, mais après que l'arbre a déjà formé son nouveau feuillage annuel : l'arbre reste donc vert en permanence. Ses fleurs sont hermaphrodites successives et mesurent de 5 à 10 mm. Le fruit, l'avocat, est en forme de poire (piriforme) ou ovoïde ou rond, de 7 à 20 cm de longueur, pesant de 100 à 1 000 g. L'avocat est, d'un point de vue botanique, une baie à un seul pépin, et non une drupe (une drupe étant un fruit indéhiscent, charnu à noyau, comme la cerise, l'abricot ou l'olive) car l'endocarpe est charnu. Il a une grosse graine centrale ovale de 3 à 5 cm de long. Le fruit de l'avocatier est principalement constitué d'eau, de pulpe, d'huile et de noyau. Les proportions de ces constituants sont, à l'instar de toutes matières naturelles et végétales, extrêmement variables. Toutefois, on admet généralement les données de composition moyennes suivantes, exprimées en pourcentage de fruit frais, données dans le tableau 1 suivant :

**Tableau 1 : Composition moyenne du fruit de l'avocatier (avocat) :**

| | |
|---|---|
| Eau | 70-85 % |
| Protéines | 1,5-4,5 % |
| Lipides | 12-23 % |
| Sucres | 1,5-5 % |
| Fibres | 1,1-1,6 % |

Par rapport à la pulpe, les protéines de l'avocat représentent 1,5 à 2,5 % (J.P. Gaillard, l'Avocatier, Ed. G.P. Maisonneuve et Larose, 1987, pp 266-67). La répartition moyenne en acides aminés, exprimée en pourcentage en poids par rapport au poids total des acides aminés, est donnée ci-dessous :

| | | | |
|---|---|---|---|
| - Alanine | 5-7 | - Lysine | 4-7 |
| - Arginine | 3-5 | - Méthionine | 1-3 |
| - Acide aspartique | 8-12 | - Phénylalanine | 4-6 |
| - Cystine-cystéine | < 1 | - Proline | 4-7 |
| - Acide glutamique | 11-13 | - Sérine | 4-6 |
| - Glycine | 4-6 | - Thréonine | 4-6 |
| - Histidine | 4-6 | - Tyrosine | 3-6 |
| - Isoleucine | 4-7 | - Valine | 4-7 |
| - Leucine | 8-11 | | |

Les principaux acides aminés présent dans l'avocat sont en général l'acide glutamique, l'acide aspartique et la leucine.

Par « peptides d'avocat » on entend les peptides naturellement présents dans toute partie d'un plant d'avocat (avocatier). Avantageusement, les peptides d'avocat sont directement obtenus à partir de n'importe quelle partie de l'avocat ou de l'avocatier, telle que la graine, le germe, les parties aériennes, le fruit, la peau, le noyau, la tige, la feuille, la fleur, l'écorce, les racines ou leur mélange. Il est aussi possible d'obtenir un extrait peptidique d'avocat à partir des co-produits de l'industrie de transformation de l'avocat, parmi lesquels on peut citer de façon non exhaustive : la pulpe fraîche d'avocat, la pulpe congelée, déshydratée, les tourteaux d'avocat issus des procédés d'extraction d'huile extraction mécanique et/ou par solvant du fruit préalablement déshydraté), les matières solides déshuilées issues des procédés d'extraction d'huile par voie humide (procédé dit de centrifugation), les matières solides déshuilées issues des procédés d'extraction d'huile d'avocat par voie enzymatique, les purées d'avocat brutes (guacamole), les déchets solides issus des unités de production de ces purées. Les peptides d'avocat sont de préférence obtenus à partir du fruit de l'avocatier (c'est-à-dire l'avocat).

Par « hamamélis » on entend un arbuste à feuillage caduc de la famille des Hamamelidaceae qui comprend notamment les espèces suivantes : *Hamamelis ovalis, Hamamelis virginiana et Hamamelis vernalis* (originaires d'Amérique du Nord), *Hamamelis japonica* (originaire du Japon), et *Hamamelis mollis* (originaire de Chine). L'hamamélis selon l'invention est de préférence plante *Hamamelis virginiana. Hamamelis virginiana* est un arbuste originaire de l'Est de l'Amérique du Nord où il se développe dans les zones humides. Il est autrement dénommé « witch hazel » c'est-à-dire « noisetier des sorciers». Par « extrait d'hamamélis », on entend un extrait directement obtenu à partir de n'importe quelle partie d'un plant ou d'un arbre d'hamamélis, telle que la graine, le germe, les parties aériennes, le fruit, la peau, le noyau, la tige, la feuille, la fleur, l'écorce, les racines ou leur mélange.

Au sens de la présente invention, on entend par « utilisation et/ou composition cosmétique » ou par « utilisation et/ou composition esthétique », une utilisation et/ou composition non pharmaceutique, c'est-à-dire qui n'est pas destinée à un usage thérapeutique et qui vise à améliorer l'esthétique et/ou le confort. L'utilisation et/ou composition cosmétique est donc de préférence effectuée/appliquée sur une partie du corps saine, en particulier la muqueuse saine, la peau saine et/ou le cuir chevelu sain. Par « ingrédient cosmétique acceptable » ou par « excipient cosmétique acceptable » ou par « véhicule cosmétique acceptable », ou encore par « ingrédient cosmétiquement acceptable » ou par « excipient cosmétiquement acceptable » ou par « véhicule cosmétiquement acceptable », on entend respectivement un ingrédient, un excipent ou un véhicule adapté à une application cosmétique notamment par voie topique ou par voie orale, non toxique, non irritant pour la peau et/ou les muqueuses et/ou le cuir chevelu, n'induisant pas de réponse allergique, qui n'est pas instable sur le plan chimique.

Par « application par voie topique » ou « application topique » on entend l'application locale directe et/ou la vaporisation de l'ingrédient sur la surface de la peau et/ou des muqueuses et/ou du cuir chevelu.

Par « muqueuse(s) » on entend la muqueuse oculaire, la muqueuse vaginale, la muqueuse uro-génitale et/ou la muqueuse buccale, notamment buccale, labiale et/ou la muqueuse gingivale, préférentiellement, les muqueuses oculaires et/ou buccales, et encore préférentiellement, la muqueuse labiale et/ou oculaire.

Par « explant » ou « explant de peau », on entend un prélèvement de cellules ou de tissu cutané, lequel peut être réalisé chez un sujet dans un but chirurgical ou pour effectuer des analyses.

En particulier, un explant peut être obtenu lors d'une exérèse chirurgicale. Par « exérèse », on entend ici une intervention chirurgicale consistant à découper (exciser) une partie plus ou moins large ou profonde de la peau pour en traiter une anomalie ou une excroissance. On procède à une exérèse soit pour retirer une tumeur cancéreuse ou suspecte de l'être, soit pour traiter une anomalie bénigne de la peau qui est gênante, que ce soit pour des raisons fonctionnelles ou esthétiques. Une exérèse inclut par exemple les échantillons de peau obtenus après chirurgie plastique (plastie mammaire, abdominale, lifting, prélèvement préputial, otoplastie, c'est-à-dire recollement d'oreille, syndactylie ou doigt surnuméraire, etc.). Un explant peut aussi être obtenu par biopsie. Par « biopsie », on entend ici un prélèvement de cellules ou tissu cutané réalisé à des fins d'analyse. Plusieurs types de procédures de biopsies sont connus et pratiqués dans le domaine. Les types les plus communs comprennent (1) la biopsie incisionnelle, dans laquelle seul un échantillon du tissu est prélevé ; (2) la biopsie excisionnelle (ou biopsie chirurgicale) qui consiste en l'ablation totale d'une masse tumorale, réalisant ainsi un geste thérapeutique et diagnostique, et (3) la biopsie à l'aiguille, dans laquelle un échantillon de tissu est prélevé avec une aiguille, celle-ci pouvant être large ou fine. D'autres types de biopsie existent, comme par exemple le frottis ou le curettage, et sont aussi englobés dans la présente invention.

Les explants comprennent au moins un type de cellules habituellement présentes dans l'hypoderme, le derme et/ou l'épiderme. Ces cellules comprennent ainsi, entre autres, des kératinocytes, des mélanocytes, des fibroblastes, des adipocytes, des cellules endothéliales, des mastocytes, des cellules de Langerhans et/ou des cellules de Merkel. De façon préférentielle, les cellules cutanées selon l'invention comprennent au moins des kératinocytes et/ou des fibroblastes. De façon plus préférentielle, les cellules cutanées selon l'invention comprennent des kératinocytes et/ou des fibroblastes.

### Composition

Dans le cadre de la présente invention, les Inventeurs ont mis en évidence, de manière tout à fait surprenante, qu'une composition comprenant un extrait de polyphénols de graines de passiflore, un extrait de peptides d'avocat et un extrait d'hamamélis permet de prévenir la formation de vergetures et de réduire les vergetures existantes sur la peau, notamment chez des femmes enceintes et post-partum, avec une grande efficacité. En particulier, les Inventeurs ont montré que, de manière surprenante, l'application d'une telle composition permet de restaurer l'organisation et l'orientation des fibres de collagène, des fibres d'élastine et des fibres oxytalanes, ainsi que les niveaux d'expression du collagène III, des cytokines de l'inflammation IL6, IL8 et IL1α, de la métalloprotéinase MMP1, et du marqueur spécifique de la vergeture alpha-Smooth Muscle Actin (αSMA). Ces données ont été confirmée par une étude menée par les Inventeurs chez des femmes enceintes et après accouchement. En effet, cette étude montre qu'une telle composition permet de prévenir la formation des vergetures et de réduire les vergetures de manière efficace, tout en étant très bien tolérée. Cette composition améliore de manière significatives les aspects inesthétiques (visuels, tactiles) des vergetures existantes ainsi que les gênes, inconforts ou incommodités associés (démangeaisons, sensations de tiraillement, sensations d'inconfort), et limite l'apparition de ces aspects inesthétiques et incommodants ou inconfortables des vergetures. Les données montrent également qu'une telle composition a effet hydratant significatif, renforçant ainsi son action anti-vergetures. Les données obtenues à partir d'un modèle de peau reproduisant de façon fidèle la physiologie de la vergeture montrent en outre que la combinaison de ces trois actifs naturels a un effet anti-vergetures supérieur à celui de chacun de ces actifs seul. En particulier, l'application de la combinaison d'un extrait de polyphénols de graines de passiflore, d'un extrait de peptides d'avocat et d'un extrait d'hamamélis conduit à une restauration significativement plus élevée des niveaux d'expression des marqueurs de l'inflammation et du marqueur spécifique de la vergeture, αSMA, à des niveaux plus proches de ceux de la peau normale non étirée. Ces résultats sont d'autant plus inattendus que les extraits de polyphénols de graines de passiflore, les extraits de peptides d'avocat et les extraits d'hamamélis n'étaient pas connus pour avoir un effet anti-vergetures.

La présente invention concerne donc une composition comprenant :
- un extrait de polyphénols de graines de passiflore ;
- un extrait de peptides d'avocat ; et
- un extrait d'hamamélis.

L'association de ces trois composants confère à la composition une action anti-vergetures remarquable, ces quatre composés agissant en synergie pour protéger la peau. Une telle composition permet d'obtenir un effet particulièrement bénéfique sur les vergetures existantes, ainsi que sur la prévention de leur apparition.

### Extrait de polyphénols de graines de passiflore

Avantageusement, la présente composition présente une teneur en polyphénols de graines de passiflore allant de 0,0025% à 10% en poids par rapport au poids total de la composition, de préférence de 0,003% à 9,5%, de préférence de 0,004% à 9%, de préférence de 0,005% à 8,5%, de préférence de 0,006% à 8%, de préférence de 0,007% à 7,5%, de préférence de 0,008% à 7%, de préférence de 0,009% à 6,5%, de préférence de 0,01% à 6%, de préférence de 0,015% à 5,5%, de préférence de 0,02% à 4,5%, de préférence de 0,025% à 4%, de préférence de 0,03% à 3,5%, de préférence de 0,035% à 3%, de préférence de 0,04% à 2,5%, de préférence de 0,045% à 2%, de préférence de 0,05% à 1,5%, de préférence de 0,055% à 1%, de préférence de 0,06% à 0,9%, de préférence de 0,065% à 0,8%, de préférence de 0,07% à 0,7%, de préférence de 0,075% à 0,6%, de préférence de 0,08% à 0,5%, de préférence de 0,085% à 0,4%, de préférence de 0,09% à 0,35%, de préférence de 0,1% à 0,3%, de préférence de 0,11% à 0,29%, de préférence de 0,12% à 0,28%, de préférence de 0,13% à 0,27%, de préférence de 0,14% à 0,26%, de préférence de 0,15% à 0,25%, de préférence de 0,16% à 0,24%, de préférence de 0,17% à 0,23%, de préférence de 0,18% à 0,22%, de préférence de 0,19% à 0,21% en poids par rapport au poids total de la composition ; de préférence encore, la teneur en polyphénols de graines de passiflore est de 0,2% en poids par rapport au poids total de la composition. Les graines de passiflore sont notamment de graines de *Passiflora incarnata* ou de *Passiflora edulis.*

Avantageusement, l'extrait de polyphénols de graines de passiflore est tel que décrit dans WO 2017/108978.

Avantageusement, l'extrait polyphénolique (ou extrait de polyphénols) de graines de Passiflore comprend donc au moins 30% en poids de polyphénols, exprimés en équivalent d'acide gallique, par rapport au poids de l'extrait sec. Cette teneur équivaut à au moins 3 mg de polyphénols par millilitre d'extrait liquide.

La teneur en polyphénols est exprimée en équivalent d'acide gallique, par rapport au poids de l'extrait sec. Ces pourcentages sont obtenus par un dosage de Folin-Ciocalteu. Dans un dosage de Folin-Ciocalteu, l'ensemble des composés phénoliques est oxydé par le réactif de Folin-Ciocalteu (disponible dans le commerce). Ce dernier comprend un mélange d'acide phosphotungstique (H3PW12040) et d'acide phosphomolybdique (H3PMo12040) qui est réduit, lors de l'oxydation des substances phénoliques, en un mélange d'oxydes bleus de tungstène (W8023) et de molybdène (Mo8023). La coloration bleue produite possède une absorption maximum aux environs de 750-760 nm. Elle est proportionnelle à la quantité de composés phénoliques oxydés. Le phénol de référence utilisé dans cette méthode est l'acide gallique (voir par exemple SINGLETON ET AL., *Colorimetry of total phenolics with phosphomolybdic-phosphotungstic acid reagents).* Les résultats obtenus par ce dosage sont donc exprimés en « % en poids de polyphénols, exprimé en équivalent d'acide gallique, par rapport au poids total de l'extrait sec ».

La teneur en polyphénols est donc un paramètre facilement mesurable pour l'homme du métier.

L'extrait de polyphénols de graines de passiflore comprend avantageusement au moins 35% en poids de polyphénols, exprimés en équivalent d'acide gallique, par rapport au poids total dudit extrait sec, c'est-à-dire au moins 3,5 mg de polyphénols par millilitre d'extrait liquide. L'extrait de polyphénols de graines de passiflore comprend plus avantageusement au moins 40% en poids de polyphénols, exprimés en équivalent d'acide gallique, par rapport au poids total dudit extrait sec, c'est-à-dire au moins 4 mg de polyphénols par ml d'extrait liquide.

Parmi les polyphénols présents dans l'extrait de polyphénols de graines de passiflore, on retrouve majoritairement les dérivés catéchiques.

Par « dérivés catéchiques » on entend les flavoinoïdes de la famille de la catéchine, également connue sous le nom de catéchol. Les dérivés catéchiques sont plus particulièrement des composés de formule générale (I) décrite dans la demande WO 2017/108978. Ainsi, les dérivés catéchiques sont plus particulièrement des composés de formule générale (I) suivante : 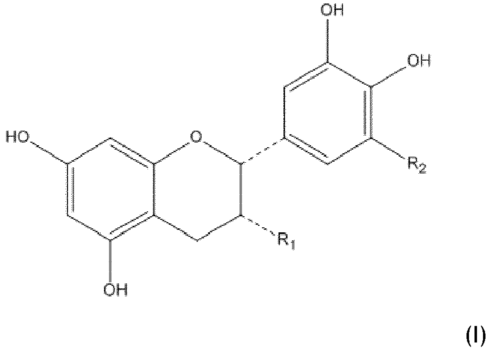 dans laquelle :
------ représente une liaison simple de configuration R ou S ;
R₁ représente OH ou un groupe galloyl de formule (II) suivante : 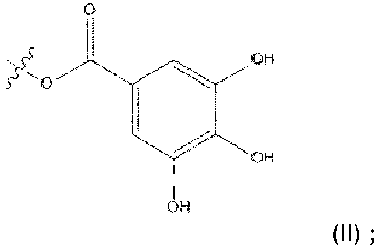 et
R₂ représente H ou OH.

Les dérivés catéchiques sont plus particulièrement des composés de formule générale (I) choisis dans le groupe constitué de :

**Tableau 2 : Formule de principaux dérivés catéchiques.**

| Nom | Configuration | R1 | R2 |
|---|---|---|---|
| (+)-catéchine | 2R, 3S | OH | H |
| (-)-épicatéchine | 3R | OH | H |
| (-)-catéchine | 3R | OH | H |
| (+)-épicatéchine | 2S, 3S | OH | H |
| (-)-épigallocatéchine | 2R, 3R | OH | OH |
| (-)-épicatéchine gallate | 2R, 3R | Groupe galloyl | H |
| (-)-épigallocatéchine gallate | 2R, 3R | Groupe galloyl | OH |
| (+)-gallocatéchine | 2R, 3S | OH | OH |
| (+)-gallocatéchine gallate | 2R, 3S | Groupe galloyl | OH |

L'extrait de polyphénols de graines de passiflore comprend avantageusement au moins 20 % en poids, en particulier au moins 24 % en poids de dérivés catéchiques, exprimés en équivalent d'acide gallique, par rapport au poids de l'extrait sec. Ainsi, dans l'extrait de polyphénols de graines de passiflore, au moins 50% en poids, en particulier au moins 60% en poids des polyphénols sont des dérivés catéchiques, exprimés en équivalent d'acide gallique, par rapport au poids de polyphénols dans l'extrait sec.

Avantageusement, l'extrait de polyphénols de graines de passiflore comprend en outre au moins 10% en poids d'acides organiques, notamment d'acide acétique, d'acide malique, d'acide citrique ou leurs mélanges, par rapport au poids de l'extrait sec.

De manière particulièrement avantageuse, l'extrait de polyphénols de graines de passiflore comprend au moins 30% en poids de polyphénols, exprimés en équivalent d'acide gallique, par rapport au poids de l'extrait sec, et au moins 10% en poids d'acides organiques, notamment d'acide acétique, d'acide malique, d'acide citrique ou leurs mélanges, par rapport au poids de l'extrait sec. Un tel extrait a pour avantage d'être riche en acides organiques, ce qui lui confère une forte activité antioxydante, anti-chélatrice et/ou hydratante.

Ainsi, selon un mode de réalisation préféré, l'extrait de polyphénols de graines de passiflore comprend :
- au moins 30% en poids de polyphénols, exprimés en équivalent d'acide gallique, par rapport au poids total de l'extrait sec de polyphénols de graines de passiflore, de préférence encore au moins 35%, de préférence encore au moins 40% en poids de polyphénols, exprimés en équivalent d'acide gallique, par rapport au poids total de l'extrait sec de polyphénols de graines de passiflore; et
- au moins 10% en poids d'acides organiques, par rapport au poids de l'extrait sec;
de préférence dans laquelle au moins 50% en poids desdits polyphénols sont des dérivés catéchiques, exprimés en équivalent d'acide gallique, par rapport au poids de polyphénols dans l'extrait sec ;
en particulier dans laquelle lesdits acides organiques sont l'acide acétique, l'acide malique, l'acide citrique ou leurs mélanges.

L'extrait de polyphénols de graines de passiflore est avantageusement obtenu par extraction solide/liquide des graines de Passiflore dans un solvant choisi parmi l'eau, les glycérols, les glycols, et leurs mélanges.

Le solvant est plus particulièrement choisi parmi les mélanges binaires eau/glycérol, eau/glycol, et leurs mélanges, avantageusement dans une proportion comprise entre 30 et 90%, en particulier entre 40 et 90 %, de préférence entre 50 % et 90 %, plus préférentiellement entre 60 % et 80 %, notamment 70 %, de glycérol et/ou de glycol dans l'eau.

De préférence, le solvant utilisé est choisi parmi les mélanges binaires eau/glycérol ou eau/propanediol, en particulier eau/propanediol, plus particulièrement eau/1,3-propanediol.

En particulier, l'extrait de polyphénols de graines de passiflore contient avantageusement en poids par rapport à l'extrait sec obtenu :
- Environ 40 % de polyphénols ;
- Environ 10 % de sucres ;
- Environ 11 % d'acides de fruits ; et
- Environ 5 % de protéines.

De manière particulière, l'extrait polyphénols de graines de passiflore ne comprend pas d'isoorientine, orientine, vitexine ou encore d'isovitexine.

Selon un mode de réalisation, l'extrait de polyphénols de graines de passiflore est obtenu par extraction solide/liquide des graines de passiflore dans un solvant choisi parmi l'eau, les glycérols, les glycols et leurs mélanges.

L'extrait de polyphénols de graines de passiflore est avantageusement obtenu par le procédé de préparation comprenant les étapes successives suivantes :
a) broyage des graines ;
b) éventuellement délipidation des graines, de préférence par pressage, extraction éthanolique ou extraction CO2 ;
c) extraction solide/liquide des graines broyées et éventuellement délipidées dans un solvant choisi parmi l'eau, les glycérols, les glycols, et leurs mélanges ;
d) séparation de la phase solide et de la phase liquide par décantation, et/ou centrifugation et/ou filtrations successives ;
e) éventuellement séchage de l'extrait obtenu à l'étape d).

L'étape a) de broyage des graines peut être réalisée par des méthodes connues de l'homme du métier, notamment à l'aide d'un broyeur à couteaux, d'un broyeur à marteaux, etc. A l'étape c), la phase d'extraction solide/liquide est réalisée de préférence à une température comprise entre 20°C et 90°C, en particulier entre 30°C et 80 °C, plus particulièrement entre 45 °C et 75°C, typiquement 70° C. La durée d'extraction est avantageusement comprise entre 30 minutes et 4h, en particulier entre 1 h et 3h, avantageusement elle est d'environ 2 heures. Avantageusement, le solvant d'extraction utilisé à l'étape c) est choisi parmi les mélanges binaires eau/glycérol, eau/glycol, et leurs mélanges, avantageusement dans une proportion comprise entre 30 et 90%, en particulier entre 40 et 90 %, de préférence entre 50 % et 90 %, plus préférentiellement entre 60 % et 80 %, notamment 70 %, de glycérol et/ou de glycol dans l'eau. En particulier, le solvant d'extraction est choisi parmi les mélanges binaires eau/glycérol ou eau/propanediol, en particulier eau/propanediol, plus particulièrement eau/1,3-propanediol.

Dans une variante avantageuse du procédé, préalablement à l'étape c), les graines de passiflore sont délipidées. Avant d'être dispersées, les graines broyées peuvent être délipidées, notamment dans de l'éthanol. L'élimination des lipides permet une meilleure efficacité des étapes d'extraction et de filtrations. Il est également et préférentiellement possible d'utiliser des tourteaux de ces graines, c'est-à-dire le résidu issu de l'extraction préalable de l'huile par solvant, technique de CO₂ supercritique par exemple et préférentiellement par pressage mécanique.

L'étape d) de séparation de la phase solide et de la phase liquide est réalisée par des méthodes connues de l'homme du métier, notamment par décantation, centrifugation et/ou filtrations successives jusqu'à parfaite limpidité et propreté microbiologique. Avantageusement, l'extrait polyphénolique tel que décrit ci-dessus peut être stabilisé par l'étape de séchage e), par des procédés connus de l'homme de l'art.

L'étape de séchage peut, par exemple, être réalisée en présence d'un support de type, par exemple, maltodextrines ou fibres d'acacia (Fibregum® société CNI). La teneur en support varie typiquement selon un ratio allant de 0% à 80% de support par rapport au pourcentage de matière sèche obtenu dans la forme liquide de l'extrait.

L'extrait est préférentiellement séché par lyophilisation afin d'obtenir une poudre finale. La poudre finale comprend avantageusement 30 à 70% en poids de matière sèche de l'extrait, le complément à 100% étant le support de lyophilisation. Plus avantageusement la poudre finale comprend 50% de matière sèche issue de l'extrait et 50% de support de lyophilisation.

Alternativement, la matière première de départ du procédé selon l'invention peut être un tourteau de graines de passiflore délipidées, notamment par pressage. Dans ce cadre et à titre d'exemple non limitatif, l'extrait polyphénolique de graines de passiflore est avantageusement obtenu peut être obtenu selon le procédé suivant :
a') mise en solution de tourteau de graines de passiflore délipidées par pressage, à 10% de matière sèche dans l'eau ;
b') extraction solide/liquide sous agitation pendant 2 heures à une température de 70°C ;
c') purification par étapes de filtrations successives ; et
d') filtration stérile.

### Extrait de peptides d'avocat

Avantageusement, la composition décrite ci-dessus présente une teneur en peptides d'avocat allant de 0,001% à 5% en poids par rapport au poids total de la composition, de préférence de 0,002% à 4%, de préférence de 0,003% à 3%, de préférence de 0,004% à 2%, de préférence de 0,005% à 1%, de préférence de 0,005% à 0,9%, de préférence de 0,006% à 0,8%, de préférence de 0,007% à 0,7%, de préférence de 0,008% à 0,6%, de préférence de 0,009% à 0,5%, de préférence de 0,01% à 0,4%, de préférence de 0,02% à 0,3%, de préférence de 0,025% à 0,25%, de préférence de 0,03% à 0,2%, de préférence de 0,04% à 0,19%, de préférence de 0,05% à 0,18%, de préférence de 0,06% à 0,17%, de préférence de 0,065% à 0,16%, de préférence de 0,07% à 0,15%, de préférence de 0,075% à 0,14%, de préférence de 0,08% à 0,13%, de préférence de 0,085% à 0,12%, de préférence de 0,09% à 0,11% en poids par rapport au poids total de la composition ; de préférence encore, la teneur en peptides d'avocat est de 0,1% en poids par rapport au poids total de la composition.

L'avocat est notamment choisi parmi les variétés *Hass, Fuerte, Ettinger, Bacon, Nabal, Anaheim, Lula, Reed, Zutano, Queen, Criola Selva, Mexicana Canta, Region Dschang, Hall, Booth, Peterson,* et *Collinson Red.*

Avantageusement, l'extrait de peptide d'avocat est tel que décrit dans WO 2010/052312. Ainsi, selon un mode de réalisation préféré, l'extrait de peptides d'avocat présente la composition suivante en acides aminés, en pourcentage de poids par rapport au poids total de l'extrait de peptides d'avocat :

| | | | |
|---|---|---|---|
| - Alanine | 6,4 - 7,8 | - Isoleucine | 4,8 - 5,8 |
| - Arginine | 4,7 - 5,7 | - Leucine | 7,6 - 9,4 |
| - Acide aspartique | 10,3 - 12,7 | - Lysine | 3,0 - 3,8 |
| - Cystine-cystéine | 2,9 - 3,5 | - Méthionine | 1,2 - 1,6 |
| - Acide glutamique | 13,0 - 15,8 | - Phénylalanine | 4,7 - 5,7 |
| - Glycine | 5,3 - 6,5 | - Proline | 4,1 - 5,2 |
| - Histidine | 2,2 - 2,6 | - Sérine | 5,5 - 6,7 |
| - Thréonine | 4,6 - 5,6 | - Valine | 5,8 - 7,2. |
| - Tyrosine | 3,6 - 4,4 | | |

L'homme du métier pourra utiliser n'importe quelle méthode de quantification des acides aminés. Il pourra notamment utiliser la méthode de mesure de la teneur en azote des peptides sous la forme de groupes alpha-aminés libres. La mesure de la teneur en azote alpha-aminé des peptides permet d'évaluer le degré d'hydrolyse des protéines ainsi que la masse molaire moyenne des peptides.

Avantageusement, les peptides d'avocat sont directement obtenus à partir de n'importe quelle partie de l'avocat ou de l'avocatier, telle que le fruit, la peau, le noyau, la feuille ou les racines de l'avocatier. Il est aussi possible d'obtenir un extrait peptidique d'avocat à partir des co-produits de l'industrie de transformation de l'avocat, parmi lesquels on peut citer de façon non exhaustive : la pulpe fraîche d'avocat, la pulpe congelée, déshydratée, les tourteaux d'avocat issus des procédés d'extraction d'huile extraction mécanique et/ou par solvant du fruit préalablement déshydraté), les matières solides déshuilées issues des procédés d'extraction d'huile par voie humide (procédé dit de centrifugation), les matières solides déshuilées issues des procédés d'extraction d'huile d'avocat par voie enzymatique, les purées d'avocat brutes (guacamole), les déchets solides issus des unités de production de ces purées. Les peptides d'avocat sont de préférence obtenus à partir du fruit de l'avocatier (c'est-à-dire l'avocat).

Selon un mode de réalisation, l'extrait de peptides d'avocat est obtenu par extraction aqueuse de fruits de l'avocatier, et délipidation. L'extrait peptidique d'avocat est donc de préférence un extrait aqueux délipidé. Cet extrait aqueux délipidé subit avantageusement une hydrolyse enzymatique. Les protéines d'avocat ont avantageusement été hydrolysées par des protéases. Selon une variante avantageuse de l'invention, les sucres présents dans l'extrait aqueux délipidé sont également hydrolysés (en présence de glucanases par exemple).

Plus particulièrement, l'extrait peptidique d'avocat est susceptible d'être obtenu par un procédé comprenant les étapes suivantes :
- obtention d'un tourteau d'avocat, avantageusement du fruit de l'avocat, par séchage puis extraction de l'huile (lipides); ensuite
- cryobroyage et délipidation complète dudit tourteau puis décantation, centrifugation et récupération du gâteau ; puis
- première hydrolyse en présence de glucanases, suivie d'une centrifugation et de l'élimination de la fraction soluble ;
- seconde hydrolyse en présence d'une ou plusieurs protéases, suivie d'une centrifugation et de l'élimination du culot ; ensuite
- concentration de la phase peptidique par nano filtration ;
- décoloration, en présence de charbon actif par exemple, suivie d'une filtration simple (10 |jm) puis d'une ultrafiltration (seuil de coupure de 10 kD) ; enfin
- le cas échéant, microfiltration stérilisante finale (0,2 µm), ajout de conservateur et conditionnement.

Selon une variante avantageuse, la première étape du procédé consiste à sécher le fruit puis à le déshuiler. Ainsi, après tranchage du fruit en fines lamelles, son séchage peut être réalisé par l'ensemble des techniques connues de l'homme de métier, parmi lesquelles on peut citer le séchage à l'air chaud, la lyophilisation ou encore le séchage osmotique. D'une façon générale, la température lors de cette étape de séchage sera avantageusement maintenue, quelle que soit la technique employée, inférieure ou égale à 80°C. Dans le cadre du présent procédé, pour des raisons de facilité de mise en œuvre et pour des raisons de coût, le séchage en séchoirs ventilés, en couche mince et sous courant d'air chaud, à une température comprise entre 70 et 75°C est préféré. La durée de l'opération peut varier de 5 à 72 heures.

Les lipides du fruit séché sont par la suite extraits soit par voie mécanique dans une presse à vis continue ou encore par voie chimique, à l'aide d'un solvant tel que l'hexane dans un extracteur de type Soxhlet ou dans un extracteur continu à bande, de type De Smet®, notamment selon le procédé décrit dans la demande FR 2 843 027, ou encore par un procédé utilisant le CO2 supercritique. Parmi les intérêts majeurs du procédé, l'huile co-produite constitue un produit bien évidemment directement valorisable. C'est pourquoi, on préfère l'extraction des lipides par voie mécanique. Le fruit sec et déshuilé, ou encore appelé tourteau, peut subir ensuite les étapes suivantes :
- cryobroyage,
- délipidation complète, notamment par un solvant alimentaire non toxique tel que l'éthanol et/ou l'acétone,
- décantation, puis lavage du tourteau à l'eau,
- centrifugation, et récupération du gâteau,
- première hydrolyse en présence d'une ou plusieurs glucanases,
- centrifugation et élimination de la fraction soluble,
- seconde hydrolyse en présence d'une ou plusieurs protéases,
- centrifugation et élimination du culot,
- concentration par nano filtration,
- décoloration en présence de charbon actif,
- filtration simple (10 |jm) puis ultrafiltration (seuil de coupure de 10 kD),
- ajout de conservateur, microfiltration stérilisante finale (0,2 |jm) et conditionnement.

L'extrait aqueux final peut contenir en poids 1 à 60 % de matière sèche, ou encore 3 à 20 % de matière sèche, de préférence 5 à 6 % de matière sèche. Par rapport au poids de la matière sèche, la teneur massique en azote alpha-aminé pourra être comprise entre 2 et 10 %, de préférence entre 5 et 7 %. Le pH d'une solution aqueuse à 1,2 % en poids d'extrait sec, sera généralement compris entre 3 et 6, plus avantageusement entre 4 et 5. Les données analytiques moyennes d'une solution aqueuse à 1,2 % en poids d'extrait sec, obtenue par un tel procédé, sont données dans le tableau 3 suivant :

**Tableau 3**

| | | |
|---|---|---|
| Azote α-aminé (méthode dite « o-phthalaldéhyde » ou « ninhydrine ») en pourcentage massique dans la matière sèche | | 4 - 10 |
| Protéines (en pourcentage massique dans la matière sèche) (N X 6,25)¹ | | 10- 30 |
| pH (dilution 1/4) | | 4,5 - 7,0 |
| Absorbance (dilution 1/4) | 420 nm | 0,1 - 0,6 |
| | 550 nm | 0,02 - 0,1 |

| | | |
|---|---|---|
| ¹ N x 6,25 correspond au dosage de l'azote total (N) d'un échantillon multiplié par un coefficient spécifique pour la protéine dosée. Quand on ne connaît pas précisément le coefficient des protéines dosées, on utilise par convention le coefficient 6,25. | | |

La composition moyenne en acides aminés de l'extrait peptidique obtenu par un tel procédé, est indiquée ci-dessous. Les valeurs sont exprimées en pourcentage en poids par rapport au poids total des acides aminés dosés. Les valeurs pour l'acide aspartique et l'acide glutamique incluent également les teneurs en asparagine et en glutamine, respectivement.

| | | | |
|---|---|---|---|
| - Alanine | 6,4 - 7,8 | - Lysine | 3,0 - 3,8 |
| - Arginine | 4,7 - 5,7 | - Méthionine | 1,2 - 1,6 |
| - Acide aspartique | 10,3 - 12,7 | - Phénylalanine | 4,7 - 5,7 |
| - Cystine-cystéine | 2,9 - 3,5 | - Proline | 4,1 - 5,2 |
| - Acide glutamique | 13,0 - 15,8 | - Sérine | 5,5 - 6,7 |
| - Glycine | 5,3 - 6,5 | - Thréonine | 4,6 - 5,6 |
| - Histidine | 2,2 - 2,6 | - Tyrosine | 3,6 - 4,4 |
| - Isoleucine | 4,8 - 5,8 | - Valine | 5,8 - 7,2. |
| - Leucine | 7,6 - 9,4 | | |

Tryptophane non dosé.

L'extrait obtenu pourra éventuellement être lyophilisé dans le but d'obtenir une poudre solide (extrait sec), mais totalement hydrosoluble par rapport aux protéines originelles de l'avocat.

Selon une variante avantageuse, 50 % au moins des peptides de l'extrait sont constitués de 10 à 30 motifs d'acides aminés. La taille de ces peptides est donc beaucoup plus petite par comparaison à celle des protéines natives de l'avocat. Ces peptides présentent donc à ce titre une bien meilleure bio disponibilité, notamment cutanée.

### Extrait d'hamamélis

Avantageusement, la composition décrite ci-dessus présente une teneur en extrait d'hamamélis allant de 1.10⁻⁴% à 50% en poids par rapport au poids total de la composition, de préférence de 3.10⁻⁴% à 45%, de préférence de 5.10⁻⁴% à 40%, de préférence de à 8.10⁻⁴% à 35%, de préférence de 0,001% à 30%, de préférence de 0,002% à 25%, de préférence de 0,003% à 20%, de préférence de 0,004% à 15%, de préférence de 0,005% à 14%, de préférence de 0,006% à 13%, de préférence de 0,007% à 12%, de préférence de 0,008% à 11%, de préférence de 0,009% à 10%, de préférence de 0,01% à 9,5%, de préférence de 0,02% à 9%, de préférence de 0,03% à 8,5%, de préférence de 0,04% à 8%, de préférence de 0,04% à 7,5%, de préférence de 0,05% à 7%, de préférence de 0,06% à 6,5%, de préférence de 0,07% à 6%, de préférence de 0,08% à 5,5%, préférence de 0,09% à 5%, de préférence de 0,1% à 4,5%, de préférence de 0,15% à 4%, de préférence de 0,2% à 3%, de préférence de 0,25% à 2%, de préférence de 0,3% à 1,9%, de préférence de 0,4% à 1,8%, de préférence de 0,5% à 1,7%, de préférence de 0,6% à 1,6%, de préférence de 0,65% à 1,5%, de préférence de 0,7% à 1,4%, de préférence de 0,8% à 1,3%, de préférence de 0,9% à 1,2%, de préférence de 0,95% à 1,15%, de préférence de 0,9% à 1,1% en poids par rapport au poids total de la composition ; de préférence encore, la teneur en extrait d'hamamélis est de 1% en poids par rapport au poids total de la composition.

L'extrait d'hamamélis peut être directement obtenu à partir de n'importe quelle partie d'un plant ou d'un arbre d'hamamélis, telle que la graine, le germe, les parties aériennes, le fruit, la peau, le noyau, la tige, la feuille, la fleur, l'écorce, les racines ou leur mélange, de préférence à partir des feuilles.

L'extrait peut alors être obtenu par les méthodes d'extraction de végétaux connues dans le domaine, par exemple par macération d'au moins une partie de la plante de préférence entre 1 et 10% (p/p) dans un solvant ou un mélange de solvants, de préférence un solvant polaire protique, et avantageusement dans l'eau, un alcool, un glycol, un polyol, un mélange eau/alcool, eau/glycol ou eau/polyol (tels que l'eau en mélange avec éthanol, glycérol, butylène glycol ou autres glycols, tel que xylitol etc.) de 100/0 à 0/100 (v/v). L'extrait est préférentiellement obtenu par extraction aqueuse.

Selon un mode de réalisation préféré, l'extrait d'hamamélis est obtenu par extraction aqueuse de feuilles d'*Hamamelis virginiana.*

L'extrait d'hamamélis est donc de préférence obtenu par extraction avec une solution aqueuse contenant plus de 60 % en poids, avantageusement au moins 70% en poids, en particulier au moins 80% en poids, plus particulièrement au moins 90% en poids, de façon particulière au moins 95% en poids, d'eau par rapport au poids total de la solution aqueuse, encore plus avantageusement ne contenant pas de butylène glycol, en particulier ne contenant pas d'alcool, plus particulièrement ne contenant que de l'eau. Selon un mode de réalisation avantageux, l'extrait selon l'invention est obtenu par extraction à une température comprise entre 0°C et 30°C, notamment par macération à froid, préférentiellement à 4°C, ou à température ambiante, c'est-à-dire entre 18 et 25°C, préférentiellement 20°C, éventuellement après une étape de séchage de la plante. Selon un mode préféré, l'extrait selon l'invention est obtenu par macération à température ambiante, préférentiellement 20°C. De manière avantageuse, l'extrait d'hamamélis n'est pas obtenu par distillation notamment à chaud et n'est pas une huile essentielle. Encore avantageusement, l'extrait d'hamamélis n'est pas obtenu à l'aide d'eau chaude ou bouillante, c'est-à-dire portée à ébullition, notamment par décoction ou par infusion.

Selon un mode de réalisation préférentielle, l'extrait d'hamamélis est obtenu par macération pendant une durée entre 30 minutes et 24 heures, préférentiellement entre 1 heure et 5 heures, encore préférentiellement 2 heures.

L'extrait obtenu est ensuite de préférence centrifugé et/ou filtré afin de récupérer la fraction soluble active, préférentiellement la fraction hydrosoluble. Il est préférentiellement filtré à un seuil de coupure de 0,45µm. Des étapes supplémentaires de décoloration et/ou désodorisation peuvent être effectuées sur l'extrait à n'importe quel stade de l'extraction et selon les techniques connues par l'homme du métier. L'extrait d'hamamélis peut également être ensuite concentré par lyophilisation ou par atomisation. De façon particulièrement avantageuse, la quantité de plante lors de l'extraction, préférentiellement les feuilles, est comprise entre 1 et 10% en poids, préférentiellement de 5 % en poids par rapport au poids total du mélange plante/solvant d'extraction, préférentiellement la solution aqueuse. En particulier la plante est broyée avant l'extraction.

De façon avantageuse la durée de l'extraction est de 2 heures et effectuée à température ambiante, préférentiellement 20°C.

L'extrait d'hamamélis obtenu est préférentiellement soluble et dissous dans un solvant notamment polaire, tel que l'eau, un alcool, un polyol, un glycol, ou un de leurs mélanges, préférentiellement un mélange hydroglycolique, encore préférentiellement contenant un glycol choisi parmi le caprylyl glycol, l'hexylène glycol, le pentylène glycol et leurs mélanges. Avantageusement l'extrait d'hamamélis est dissous dans une solution aqueuse contenant du pentylène glycol et/ou du caprylyl glycol, en particulier contenant entre 0,01 et 10 % en poids de pentylène glycol et/ou de caprylyl glycol par rapport au poids total la solution aqueuse, plus particulièrement entre 0,1 et 5 % en poids du pentylène glycol et/ou du caprylyl glycol par rapport au poids total la solution aqueuse.

Selon un mode de réalisation avantageux, l'extrait d'hamamélis est LYS'SUN®, commercialisé par la société BASF (décrit notamment dans EP3054963).

### Ingrédients additionnels et forme de la composition

Selon un mode de réalisation, la composition décrite ci-dessus comprend au moins un ingrédient cosmétique ou actif cosmétique additionnel.

On peut notamment ajouter le beurre de Karité, l'huile d'avocat (par exemple telle que décrite dans WO2007/057439), le glycérol et l'alpha-tocophérol.

D'autres composés peuvent avantageusement être ajoutés à ladite composition, tels que le panthénol dextrogyre le lactate méthysilanol ou des oligoéléments à base de cuivre et zinc, tels que le gluconate de zinc et le gluconate de cuivre.

La composition peut en outre comprendre au moins un composé choisi dans le groupe constitué par les agents anti-irritant et/ou apaisants et/ou anti-vieillissement et/ou hydratants et/ou les actifs promoteurs de réparation cutanée.

Les agents anti-irritants et/ou apaisants et/ou anti-vieillissement et/ou hydratants et/ou les actifs promoteurs de réparation cutanée pouvant être utilisés dans ce cadre sont avantageusement la glycine, les sucres et/ou peptides végétaux dont le lupin, le lupéol (FR 2 822 821, FR 2 857 596), les oxazolines (WO 04/112741, WO 2006/114443), les oxazolidinones (WO 04/05005), l'acide lipoïque, l'alpha bisabolol, les dérivés de réglisse, l'enoxolone, l'ectoïne, les furanes d'avocat (pouvant être obtenus par le procédé décrit dans la demande internationale WO 01/21605, par exemple l'Avocadofurane®), les extraits de Centella asiatica en particulier l'acide madécassique ou l'acide asiatique, la caféine, le rétinol, le rétinal, l'acide rétinoïque, l'oxyde de zinc, le magnésium, le silicium, le cuivre, le zinc, le manganèse, le sélénium, l'acide hyaluronique, l'acide azélaïque et ses sels ou esters, l'acide salicylique et ses dérivés, l'alpha hydroxyacide (AHA), les esters d'AHA, l'acide pyrrolidone carboxylique et dérivés, les céramides, le cholesterol, le squalane, les phospholipides, le beta carotène, la vitamine A, la vitamine E, la vitamine C, la vitamine B3 (niacinamide, nicotinamide), la vitamine B5 (panthenol), la vitamine B6, le peroxyde de benzoyle, l'urée, la coenzyme Q10, la glucosamine et ses sels, la N-acétyl glucosamine, l'arabinogalactane, les eaux de source ou thermales (eau d'Avène, eau de la Roche Posay, eau de Saint Gervais, eau d'Uriage, eau de Gamarde), les peptides de soja, les insaponifiables d'huile de sésame (par exemple la Sésaline®), les extraits de Maca (notamment tels que décrits dans WO2004112742, par exemple le Skinergium®), les hydrolysats de Schizandra (notamment tels que décrits dans WO2011012612 et WO2011012615, par exemple le Sweetone®), les extraits d'Acacia (notamment tels que décrits dans WO2011064402, par exemple l'AQUALICIA®), l'huile de Lupin (notamment telle que décrite dans WO9847479, par exemple l'Alpha-Lupaline®), les extraits peptidiques de la microalgue *Chlamydomonas acidophila* (notamment tels que décrits dans FR1874321, par exemple l'Algaenia®), les extraits de Kapokier riches en polyphénols (notamment tels que décrit dans FR1763152), le concentrat de Maracuja (notamment tel que décrit dans WO2015/044254 A1, par exemple la Passioline®), et un acide hyaluronique de moyen poids moléculaire. Les actifs promoteurs de réparation cutanée sont avantageusement choisis parmi le concentrat de Maracuja et un acide hyaluronique de moyen poids moléculaire, reconnus pour leurs propriétés réparatrices. Les oxazolines pouvant être utilisées dans la présente composition sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide®.

La présente composition peut également comprendre des sucres d'avocat, des agents restructurant de la barrière cutanée, des composés antifongiques, des conservateurs antiseptiques et des composés contenant des insaponifiables d'huiles végétales, les peptides de lupin (tels que ACTIMP®), l'huile d'avocat, le butyl avocadate, les cyclocéramides, la génistéine, les concentrats de colza, et les concentrats de maïs.

Les sucres d'avocats sont avantageusement le D-mannoheptulose et/ou le perséitol. Les sucres d'avocats correspondent plus avantageusement à l'extrait hydrosoluble de sucres d'avocat décrit dans la demande WO 2005/115421. Ainsi, les sucres d'avocats sont de préférence choisis parmi les sucres en C7 ou dérivés de formule (III) suivante 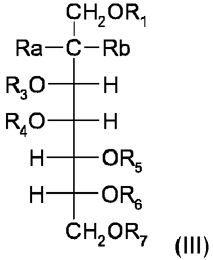 dans laquelle :
Ra représente un atome d'hydrogène et Rb représente un -OR₂ ou CRaRb représente le radical CO ;
R₁, R₂, R₃, R₄, R₅, R₆ et R₇ représentent, indépendamment l'un de l'autre :
   - un atome d'hydrogène ou
   - un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy (-OC₂H₅) et groupement -SO₃M avec M représentant un atome d'hydrogène, un ion ammonium NH₄⁺ ou un ion métallique ; ou
   - un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy (-OC₂H₅) et groupement -SO₃M avec M représentant un atome d'hydrogène, un ion ammonium NH₄⁺ ou un ion métallique.

Les agents restructurant de la barrière cutanée permettent de stimuler la synthèse des lipides clés de l'épiderme et sont avantageusement des concentrats de tournesol, encore plus avantageusement des concentrats de tournesol linoléiques, tels que l'actif commercialisé par les Laboratoires Expanscience, Soline® (cf. la demande WO 01 /21150), des insaponifiables d'huile végétale, tel que l'Avocadofurane® (cf. la demande WO 01/21150), et des agonistes de PPARs (rosiglitazone, pioglitazone).

Les composés antifongiques pouvant être utilisés dans la présente composition sont avantageusement l'econazole et le ketoconazole.

Les conservateurs antiseptiques pouvant être utilisés dans la présente composition sont par exemple le triclosan, la chlorhéxidine, les ammoniums quaternaires.

Les composés contenant des insaponifiables d'huiles végétales pouvant être utilisés dans le cadre de la présente invention sont avantageusement choisis dans le groupe constitué par les lipides furaniques d'avocat, les insaponifiables d'avocat, les insaponifiables de soja, les insaponifiables d'avocat et de soja, les concentrats d'huile de lupin, les concentrats d'huile de tournesol et leurs mélanges.

Les lipides furaniques d'avocat pouvant être utilisés dans la présente composition sont avantageusement des 2-alkyl furanes naturels, notamment l'actif Avocadofurane® commercialisé par les Laboratoires Expanscience, pouvant être obtenus par le procédé décrit dans la demande internationale WO 01/21605.

Les insaponifiables d'avocat et de soja pouvant être utilisés dans la présente composition sont avantageusement un mélange d'insaponifiables d'avocat furanique et d'insaponifiables de soja, dans un rapport respectif d'environ 1 /3-2/3. Les insaponifiables d'avocat et de soja sont encore plus avantageusement le produit Piasclédine®, commercialisé par les Laboratoires Expanscience.

Les concentrats d'huile de lupin pouvant être utilisés dans la présente composition sont avantageusement des concentrats obtenus par distillation moléculaire d'huile de lupin, avantageusement d'huile de lupin blanc doux, tels que ceux décrits dans la demande internationale WO 98/47479. Ils contiennent avantageusement environ 60% en poids d'insaponifiables.

Les concentrats d'huile de tournesol pouvant être utilisés dans la présente composition sont avantageusement des concentrats de tournesol linolé linoléiques, tels que l'actif commercialisé par les Laboratoires Expanscience, Soline® (cf. la demande internationale WO 01/21150).

Selon un aspect préféré, la composition comprend à la fois un extrait de polyphénols de graines de passiflore, un extrait de peptides d'avocat et un extrait d'hamamélis tels que définis ci-dessus, et au moins un actif choisi parmi le beurre de Karité, l'huile d'avocat, le glycérol et l'alpha-tocophérol et leurs mélanges.

L'association de ces composants confère à la composition une action anti-vergetures remarquable, ces quatre composés agissant en synergie pour protéger la peau. Une telle composition permet d'obtenir un effet particulièrement bénéfique sur les vergetures existantes, ainsi que sur la prévention de leur apparition, comme l'ont montré les Inventeurs.

Selon un mode de réalisation avantageux, la composition se présente sous une forme propre à une administration par voie topique ou par voie orale. Avantageusement, la composition comprend en outre au moins un excipient cosmétiquement acceptable. Dans ce cas, la composition est une composition cosmétique et/ou esthétique, qui est de préférence topique ou orale.

Ladite composition topique peut comprendre en outre au moins un excipient cosmétiquement acceptable et/ou au moins un véhicule approprié qui peut être tout excipient et/ou tout véhicule parmi ceux connus de l'homme du métier en vue d'obtenir une composition cosmétique telle que définie ci-dessus, en particulier sous forme d'une crème, d'une lotion, d'un gel, d'un spray, d'un patch, d'une eau, d'une pommade, de lait ou d'huile, d'une émulsion, avec en outre des composants connus de l'homme du métier pour améliorer, modifier ou stabiliser la composition d'un point de vue cosmétique ou esthétique.

Pour l'ingestion par voie orale, de nombreuses formes de réalisation et notamment des compléments alimentaires sont possibles. Leur formulation est réalisée par les procédés usuels pour produire des comprimés, des gélules, des capsules molles, des dragées, des émulsions, des gels. En particulier, le mélange d'extrait de polyphénols de graines de passiflore, d'extrait de peptides d'avocat et d'extrait d'hamamélis tels que définis ci-dessus, et les éventuels autres ingrédients de la composition orale peuvent être incorporés dans toutes formes de compléments alimentaires ou d'aliments enrichis, par exemple, des barres alimentaires, des poudres compactées ou non, des boissons, des produits laitiers et en particulier des yaourts et des yaourts à boire. Les poudres peuvent être diluées dans de l'eau, des sodas, des jus de fruits, des produits laitiers ou à base de soja, de riz, ou être incorporées dans des barres alimentaires. La composition orale peut également comprendre au moins un excipient cosmétiquement acceptable et/ou au moins un véhicule approprié qui peut être tout excipient et/ou tout véhicule parmi ceux connus de l'homme du métier en vue d'obtenir une composition cosmétique orale.

Les conditions opératoires pour préparer ces compositions cosmétiques font partie des connaissances générales de l'homme du métier.

Selon un mode de réalisation alternatif, la composition d'intérêt comprend en outre au moins un excipient pharmaceutiquement acceptable. Dans ce cas, la composition est une composition pharmaceutique. La composition pharmaceutique selon l'invention se présente avantageusement sous une forme propre à une administration par voie topique ou par voie orale. Ladite composition pharmaceutique peut également comprendre au moins un excipient pharmaceutiquement acceptable et/ou au moins un véhicule approprié qui peut être tout excipient et/ou tout véhicule parmi ceux connus de l'homme du métier en vue d'obtenir une composition pharmaceutique utilisable selon l'invention, avec en outre des composants connus de l'homme du métier pour améliorer, modifier ou stabiliser la composition d'un point de vue pharmaceutique.

Les conditions opératoires pour préparer ces compositions pharmaceutiques selon l'invention font partie des connaissances générales de l'homme du métier.

### Utilisation cosmétique et/ou esthétique et méthode de soin cosmétique non thérapeutique et/ou esthétique de la peau

Les Inventeurs ont montré, de manière surprenante, qu'une composition cosmétique comprenant un extrait de polyphénols de graines de passiflore, un extrait de peptides d'avocat et un extrait d'hamamélis permet de prévenir la formation de vergetures et de réduire les vergetures existantes sur la peau, notamment chez des femmes enceintes et post-partum, avec une grande efficacité. En particulier, les Inventeurs ont montré que, de manière surprenante, l'application d'une telle composition permet de restaurer l'organisation et l'orientation des fibres matricielles du derme et en particulier des fibres élastiques de la peau ainsi que les niveaux d'expression de marqueurs de l'organisation des fibres matricielles du derme et en particulier des fibres élastiques, du marqueur spécifique de la vergeture alpha-Smooth Muscle Actin (αSMA) et de marqueurs de l'inflammation. Il en résulte un effet anti-vergetures démontré in vivo, en particulier chez des femmes enceintes et après accouchement. La présente composition améliore de manière significative les aspects inesthétiques (visuels, tactiles) des vergetures existantes ainsi que les gênes, inconforts ou incommodités associés (démangeaisons, sensations de tiraillement, sensations d'inconfort), et limite l'apparition de ces aspects inesthétiques et incommodants ou inconfortables des vergetures, tout en étant très bien tolérée.

La mise au point de cette composition cosmétique ayant une grande efficacité anti-vergetures a notamment reposé sur l'utilisation d'un nouveau modèle de peau, capable de reproduire les différentes caractéristiques physiologiques de la peau vergeturées, notamment des vergetures des femmes enceintes.

Ce modèle de peau utilisé par les Inventeurs est obtenu en plaçant des cellules cutanées (cellules de peau) sous contrainte mécanique afin de modéliser l'étirement de la peau, notamment au cours de la grossesse.

Avantageusement, les cellules cutanées ont été obtenues à partir d'un échantillon cutané.

Par « échantillon cutané », on entend ici tout échantillon contenant des cellules de la peau. Les échantillons cutanés comprennent donc aussi bien les explants de peau, que les cultures de cellules cutanées en suspension, les cultures de cellules cutanées en monocouche, les cultures de cellules cutanées en bicouche et les modèles tissulaires, dont les cultures de peaux reconstruites (ou modèle de peau reconstruite) et les cultures de muqueuses reconstruites. Avantageusement, les cellules cutanées utilisées dans le modèle de peau sont choisies parmi les cultures de peau reconstruite et les explants de peau. Les cellules cutanées utilisées dans le modèle de peau sont préférentiellement des explants de peau.

Dans le présent modèle de peau, les cellules cutanées sont placées dans des conditions de culture appropriées (notamment dans des conditions de survie) pendant le temps désiré (en général plusieurs jours, par exemple entre 2 et 28 jours, avantageusement entre 3 et 10 jours) entre deux mors, avec ou sans contrainte mécanique à force constante (avantageusement une force constante uni-axiale, comprise entre 0,1 et 2 Newton, préférentiellement 1N, jusqu'à 50% d'allongement

Le modèle utilisé permet de reproduire fidèlement la physiologie de la vergeture. Il a notamment été montré que la matrice extra cellulaire dermique subissait des modifications similaires à celles observées dans des zones de vergetures *in vivo* (orientation des fibres de collagène et d'élastine dans le sens de la tension ; altération des fibres oxytalanes sous la jonction dermo-épidermique ; diminution de l'expression du collagène III au niveau des fibres réorientées ; induction d'une inflammation via le relargage de cytokines (IL6, IL8, IL1α)). Une telle tension mime donc l'étirement de la peau causant les vergetures, notamment au cours de la grossesse.

Un tel modèle de peau permet donc avantageusement d'étudier la physiologie de la vergeture et d'identifier des actifs ou des formulations permettant de prévenir et de traiter les vergetures.

Ainsi, la présente invention concerne l'utilisation d'une composition telle que définie dans la section précédente, pour prévenir l'apparition des vergetures sur la peau et/ou réduire les vergetures sur la peau, en particulier pendant l'adolescence et/ou pendant la grossesse. En particulier, la présente invention concerne l'utilisation cosmétique et/ou esthétique d'une composition cosmétique telle que définie dans la section précédente, pour prévenir l'apparition des vergetures sur la peau et/ou réduire les vergetures sur la peau, en particulier pendant l'adolescence et/ou pendant la grossesse.

La présente invention concerne en outre une méthode de soin cosmétique et/ou esthétique de la peau, pour prévenir l'apparition des vergetures sur la peau et/ou réduire les vergetures sur la peau, en particulier pendant l'adolescence et/ou pendant la grossesse, comprenant l'administration d'une composition cosmétique telle que définie dans la section précédente.

Ainsi, la présente utilisation cosmétique ou la présente méthode de soin cosmétique permet de prévenir et/ou réduire les effets esthétiques et/ou cosmétiques indésirables des vergetures tels que définis ci-dessus. Ces effets comprennent notamment le changement de teinte et/ou de relief de la peau, l'étirement et/ou la distension de la peau, la perte (et/ou la diminution) de fermeté et/ou de l'élasticité et/ou de souplesse de la peau, ainsi que les gênes, inconforts ou incommodités associés (démangeaisons, sensations de tiraillement, sensations d'inconfort). Ainsi, l'invention concerne l'utilisation cosmétique et/ou esthétique, ainsi qu'une méthode de soin cosmétique et/ou esthétique, pour prévenir et/ou réduire les effets esthétiques et/ou cosmétiques indésirables des vergetures, tels que définis ci-dessus, par exemple tels que le changement de teinte et/ou de relief de la peau (notamment la rugosité, l'étirement et/ou la distension de la peau, la diminution ou la perte de fermeté et/ou la perte de l'élasticité, ainsi que les gênes, inconforts ou incommodités associés (démangeaisons, sensations de tiraillement, sensations d'inconfort).

La présente utilisation cosmétique ou la méthode de soin cosmétique est donc de préférence une utilisation ou une méthode pour améliorer les aspects des vergetures de la peau, en particulier les aspects esthétiques, tels que le changement de teinte et/ou de relief de la peau, l'étirement et/ou la distension de la peau, la perte (et/ou la diminution) de fermeté et/ou de l'élasticité et/ou de souplesse, ainsi que les gênes, inconforts ou incommodités associés (démangeaisons, sensations de tiraillement, sensations d'inconfort).

Avantageusement, la présente utilisation cosmétique ou la méthode de soin cosmétique permet de préserver, d'améliorer et/ou de restaurer l'organisation et l'orientation des fibres matricielles du derme et en particulier des fibres élastiques de la peau (notamment les fibres de collagène, les fibres d'élastine et les fibres oxytalanes) ainsi que les niveaux d'expression de marqueurs de l'organisation des fibres matricielles du derme et en particulier des fibres élastiques (tels que le collagène I, le collagène III, l'élastine, la fibrilline 1), du marqueur spécifique de la vergeture alpha-Smooth Muscle Actin (αSMA) et de marqueurs de l'inflammation (tels que les interleukines IL6, IL8 et IL1α, et la métalloprotéinase MMP1). Selon un mode de réalisation particulièrement préféré, la présente utilisation cosmétique ou la méthode de soin cosmétique pour réduire les vergetures comprend, consiste essentiellement en, ou consiste en, toute amélioration de l'état physiologique de la peau ou de son apparence, notamment au niveau des vergetures, de préférence par atténuation d'au moins une des caractéristiques des vergetures telles que définies ci-dessus, de préférence comparativement à la peau normale telle que définie ci-dessus (avantageusement la peau du sujet considéré, située au niveau des zones non vergeturées). Avantageusement, la réduction des vergetures comprend, consiste essentiellement en, ou consiste en ce qu'au moins l'un des critères de réduction des vergetures, tels que définis ci-dessus, est rempli. Selon un mode de réalisation particulièrement préféré, la présente utilisation cosmétique ou la méthode de soin cosmétique pour prévenir les vergetures comprend, consiste essentiellement en, ou consiste en toute limitation de l'apparition et/ou du développement d'au moins une des caractéristiques des vergetures telles que définies ci-dessus, de préférence comparativement à la peau normale telle que définie ci-dessus (avantageusement la peau du sujet considéré, située au niveau des zones non vergeturées). Avantageusement, la prévention des vergetures comprend, consiste essentiellement en, ou consiste en ce qu'au moins l'un des critères de prévention des vergetures, tels que définis ci-dessus, est rempli.

Les méthodes d'évaluation de chacun des critères sont connues de l'homme du métier. L'homme du métier saura définir les conditions expérimentales permettant d'évaluer ces critères et il pourra notamment s'appuyer sur l'étude clinique décrite ci-dessous.

Par exemple, la taille des vergetures peut être déterminée en mesurant la longueur et la largeur des vergetures. Les modifications de la taille des vergetures pourront être évaluées en comparant la taille des vergetures sur une zone de la peau avant et après application de la présente composition, selon un intervalle de temps déterminé, ou encore en comparant à une échelle déterminée.

Le nombre de vergetures peut être déterminé en comptant les vergetures individuelles visibles à l'œil nu sur une zone de peau (nombre de vergetures par unité de surface de peau, en cm²). Les modifications du nombre de vergetures pourront être évaluées en comparant le nombre de vergetures sur une zone de la peau avant et après application de la présente composition, selon un intervalle de temps déterminé, ou encore en comparant à une échelle déterminée.

La couleur des vergetures peut être évaluée à l'aide d'un chromatomètre ou d'un colorimètre.

Les paramètres viscoélastiques de la peau (étirement, distension, fermeté, élasticité) peuvent être évalués à l'aide d'un cutomètre.

Le relief de la peau pourra être évalué par toute méthode connue de l'homme du métier. Les méthodes décrites dans WO 2014/020173 ou dans WO 2015/173413.

Les paramètres physiologiques propres aux vergetures, tels que définis ci-dessus, peuvent être évalué comme décrit dans les exemples ci-dessous. Notamment, l'organisation et/ou l'orientation et/ou l'état des fibres de collagène, des fibres d'élastine et des fibres oxytalanes, l'épaisseur de la jonction dermo-épidermique (JDE) peut être évaluée à partir d'un échantillon cutané prélevé chez le sujet et observé par microscopie (par des techniques de prélèvement d'échantillons cutanés et de microscopie connues de l'homme du métier).

Les niveaux d'expression du gène de la collagène I, du gène de la collagène III (de préférence les niveaux d'expression du collagène III sont maintenus), du marqueurαSMA et des marqueurs de l'inflammation tels que l'IL6, IL8, IL1α, TNF-alpha et la métalloprotéinase MMP1 peuvent être évalués par toute méthode connue de l'homme du métier.

Le terme « niveau d'expression » se réfère à la concentration cellulaire dudit marqueur. Si le marqueur est un gène, le « niveau d'expression » correspond à la concentration cellulaire d'au moins un des produits du gène dudit marqueur. Plus précisément, le niveau d'expression du gène correspond à la quantité ou à la concentration cellulaire du transcrit dudit gène ou de la protéine issue dudit transcrit. Les niveaux d'expression d'un gène peut être mesurée par exemple au niveau nucléotidique, en mesurant la quantité de transcrits dudit gène, et peut aussi être mesurée par exemple au niveau peptidique, en mesurant par exemple la quantité des protéines issus desdits transcrits. Ainsi, par « mesure du niveau d'expression d'un gène » on entend ici la mesure de la quantité ou de la concentration cellulaire de produit du gène sous sa forme peptidique ou sous sa forme nucléotidique. De façon générale, l'expression du marqueur biologique selon l'invention sera détectée in vitro à partir d'un échantillon de peau.

La méthode de mesure des niveaux d'expression d'un gène peut comprendre l'extraction d'un échantillon d'ARNm (ou de l'ADNc correspondant) ou d'un échantillon de protéine. Celui-ci peut ensuite être directement utilisé pour mesurer l'expression du marqueur. La préparation ou l'extraction d'ARNm (ainsi que la rétrotranscription de celui-ci en ADNc), ou de protéines, à partir d'un échantillon de cellules de peau ne sont que des procédures de routine bien connues de l'homme du métier. Une fois qu'un échantillon d'ARNm (ou d'ADNc correspondant) ou de protéine est obtenu, l'expression du marqueur, au niveau soit des ARNm (c'est-à-dire dans l'ensemble des ARNm ou des ADNc présents dans l'échantillon), soit des protéines (c'est-à-dire dans l'ensemble des protéines présentes dans l'échantillon), peut être mesurée. La méthode utilisée pour ce faire dépend alors du type de transformation (ARNm, ADNc ou protéine) et du type d'échantillon disponible. Quand l'expression du marqueur est mesurée au niveau de l'ARNm (ou d'ADNc correspondant), n'importe quelle technologie habituellement utilisée par l'homme du métier peut être mise en œuvre. Ces technologies d'analyse du niveau d'expression des gènes, comme par exemple l'analyse du transcriptome, incluent des méthodes bien connues telles que la PCR (Polymerase Chain Reaction, si on part d'ADN), la RT-PCR (Reverse Transcription-PCR, si on part d'ARN) ou la RT-PCR quantitative ou encore les puces d'acides nucléiques (dont les puces à ADN et les puces à oligonucléotides) pour un plus haut débit.

Par « puces d'acides nucléiques », on entend ici plusieurs sondes d'acides nucléiques différentes qui sont attachées à un substrat, lequel peut être une micropuce, une lame de verre, ou une bille de la taille d'une microsphère. La micropuce peut être constituée de polymères, de plastiques, de résines, de polysaccharides, de silice ou d'un matériau à base de silice, de carbone, de métaux, de verre inorganique, ou de nitrocellulose.

Les sondes peuvent être des acides nucléiques tels que les ADNc (« puce à ADNc »), les ARNm (« puce à ARNm ») ou des oligonucléotides (« puce à oligonucléotides »), lesdits oligonucléotides pouvant typiquement avoir une longueur comprise entre environ 25 et 60 nucléotides.

Pour déterminer le profil d'expression d'un gène particulier, un acide nucléique correspondant à tout ou partie dudit gène est marqué, puis mis en contact avec la puce dans des conditions d'hybridation, conduisant à la formation de complexes entre ledit acide nucléique cible marqué et les sondes attachées à la surface de la puce qui sont complémentaires de cet acide nucléique. La présence de complexes hybridés marqués est ensuite détectée.

Ces technologies permettent de suivre le niveau d'expression d'un gène en particulier ou de plusieurs gènes voire même de tous les gènes du génome (full genome ou full transcriptome) dans un échantillon biologique (cellules, tissus...). Ces technologies sont utilisées en routine par l'homme du métier et il n'est donc pas besoin de les détailler ici. Des exemples de mises en œuvre de l'invention basées sur l'analyse d'expression génique (puces à ADNc) et sur la PCR quantitative sont décrits dans la section expérimentale. Alternativement, il est possible d'utiliser toute technologie actuelle ou future permettant de déterminer l'expression des gènes sur la base de la quantité d'ARNm dans l'échantillon. Par exemple, l'homme du métier peut mesurer l'expression d'un gène par hybridation avec une sonde d'acide nucléique marquée, comme par exemple par Northern blot (pour l'ARNm) ou par Southern blot (pour l'ADNc), mais aussi par des techniques telles que la méthode d'analyse sérielle de l'expression des gènes (SAGE) et ses dérivés, tels que LongSAGE, SuperSAGE, DeepSAGE, etc. Il est aussi possible d'utiliser des puces à tissu (aussi connues en tant que TMAs : « tissue microarrays »). Les tests habituellement employés avec les puces à tissu comprennent l'immunohistochimie et l'hybridation fluorescente in situ. Pour l'analyse au niveau de l'ARNm, les puces à tissu peuvent être couplées avec l'hybridation fluorescente in situ. Enfin, il est possible d'utiliser le séquençage massif en parallèle pour obtenir déterminer la quantité d'ARNm dans l'échantillon (RNA-Seq ou « Whole Transcriptome Shotgun Sequencing »). À cet effet, plusieurs méthodes de séquençage massif en parallèle sont disponibles. De telles méthodes sont décrites dans, par exemple, US 4,882,127 ; US 4,849,077 ; US 7,556,922 ; US 6,723,513 ; WO 03/066896 ; WO 2007/111924 ; US 2008/0020392 ; WO 2006/084132 ; US 2009/0186349 ; US 2009/0181860 ; US 2009/0181385; US 2006/0275782; EP-B1-1141399 ; Shendure & Ji, Nat Biotechnol., 26(10) : 1135-45, 2008 ; Pihlak et al., Nat Biotechnol., 26(6) : 676- 684, 2008 ; Fuller et al., Nature Biotechnol., 27(11) : 1013-1023, 2009 ; Mardis, Genome Med., 1(4): 40, 2009 ; Metzker, Nature Rev. Genet., 11(1) : 31-46, 2010.

Quand l'expression du marqueur est mesurée au niveau protéique, il est possible d'employer des anticorps spécifiques, en particulier dans des technologies bien connues telles que l'immunoprécipitation, l'immunohistologie, le western blot, le dot blot, l'ELISA ou l'ELISPOT, les puces à protéines, les puces à anticorps, ou les puces à tissu couplées à l'immunohistochimie. Parmi les autres techniques qui peuvent être utilisées sont comprises les techniques de FRET ou de BRET, la cytométrie de flux, les méthodes de microscopie ou d'histochimie, dont notamment les méthodes de microscopie confocale, de microscopie à fluorescence, de microscopie électronique, de microscopie à force atomique, les méthodes basées sur l'utilisation d'une ou plusieurs longueurs d'onde d'excitation et d'une méthode optique adaptée, comme une méthode électrochimique (les techniques voltamétrie et d'ampérométrie), et les méthodes de radiofréquence, comme la spectroscopie résonance multipolaire, confocale et non-confocale, comme la détection de fluorescence, luminescence, chemiluminescence, absorbance, réflectance, transmittance, biréfringence ou index de réfraction (par exemple, par résonance des plasmons de surface, ou « surface plasmon resonance » en anglais, par ellipsométrie, par méthode de miroir résonnant, etc.), l'imagerie par résonance radioisotopique ou magnétique, l'analyse par électrophorèse en gel de polyacrylamide (SDS-PAGE); par électrophorèse bidimensionnelle, par spectrophotométrie, par spectrométrie de masse et spectrométrie de masse en tandem, par chromatographie liquide ou gazeuse couplée à la spectrométrie de masse ou à la spectrométrie de masse en tandem. Toutes ces techniques sont bien connues de l'homme du métier et il n'est pas nécessaire de les détailler ici. Le collagène fait partie d'une famille de protéines dites structurales, le plus souvent présente sous forme fibrillaire. Elle est présente dans la matrice extracellulaire des organismes animaux. Ces protéines ont pour fonction de conférer aux tissus une résistance mécanique à l'étirement. Chaque type de collagène possède une structure propre et se retrouve dans des organes particuliers. Par exemple, le collagène de type I (collagène I) intervient dans la formation de la peau, des tendons, des os et de la cornée, tandis que le type III se retrouve principalement au niveau du système cardiovasculaire (collagène III). Le collagène I humain possède une chaine alpha1, dont la séquence protéique est représentée par la séquence de référence GenBank: EAW94631.1 ou GenBank: EAW94630.1 (NCBI), codée par le gène COL1A1 (référence NCBI : GeneID:1277) et une chaine alpha2 , dont la séquence protéique est représentée par la séquence de référence GenBank: EAW76796.1 ou GenBank: EAW76795.1, codé par le gène COL1A2 (référence NCBI : GeneID:1278).

Le collagène III humain possède une séquence protéique représentée par la séquence de référence GenBank: EAX10911.1, ou GenBank: AGL34959.1, ou GenBank: ACZ58371.1, codée par le gène COL3A1 (référence NCBI : GeneID:1281).

La fibrilline 1 est une glycoprotéine, composante essentielle du réseau élastique du derme. Les fibres oxytalanes, allant jusqu'au contact de la jonction dermo-épidermique, sont des fibres très riches en fibrilline-1 recouvrant des fonctions essentielles dans le maintien de l'intégrité et des propriétés mécaniques de la peau. La fibrilline 1 humaine possède une séquence protéique représentée par la séquence de référence GenBank: BAD16738.1, ou GenBank: BAD16739.1, ou GenBank: BAD16737.1, ou GenBank: AAI46855.1, ou GenBank: ACZ58372.1, codée par le gène FBN1 (référence NCBI : GeneID:2200).

L'élastine est une protéine du tissu conjonctif responsable des propriétés élastiques de la peau ; elle est la composante majeure du réseau élastique de la peau. L'élastine humaine possède une séquence protéique représentée par la séquence de référence GenBank: AAC98395.1, ou GenBank: AAC98394.1, ou GenBank: AAC98393.1, codée par le gène ELN (référence NCBI : GeneID:2006).

Les marqueurs l'inflammation sont bien connus de l'homme du métier. Ils comprennent notamment des cytokines telles que TNFα ou les interleukines, y compris IL1α, IL-1RA, IL-2, IL-6, IL-8, et des métalloprotéases matricielles telles que MMP1 et MMP3. Préférentiellement, le marqueur de l'inflammation est choisi dans le groupe constitué par les interleukines, de préférence IL1 α, IL6 et IL8, et par TNFα et MMP1 L'interleukine IL1α humaine possède une séquence protéique représentée par la séquence de référence NCBI : NP_000566. Cette protéine est codée par le gène IL1A humain (référence NCBI : Gene ID: 3552), dont la séquence correspond à la référence NCBI : NM_000575. L'interleukine IL1α est le marqueur inflammatoire primaire de la cascade inflammatoire. Cette cytokine sécrétée par de nombreuses cellules en réponse à des stress inflammatoires. Elle est notamment exprimée constitutivement par les kératinocytes, dans le cytoplasme desquels elle est stockée. Elle peut être relarguée passivement en réponse à un stress aigu, tell qu'une altération de la membrane. Cependant, un stress plus modéré peut provoquer en outre une augmentation de la synthèse de cette cytokine.

Les interleukines IL-6 et IL-8 humaines sont des cytokines pro-inflammatoires. La séquence protéique de la première correspond à la séquence de référence NCBI : NP_000591.1. Cette protéine est codée par le gène IL6 humain (référence NCBI : Gene ID : 3569). La séquence de celui-ci est accessible sous la référence NCBI : NM_000600. La protéine IL-8, quant à elle, possède une séquence correspondant à une référence NCBI choisie parmi : NP_001230140.1, NP_001553.1 et NP_001230140.1. Cette protéine est codée par le gène IL8 humain (référence NCBI : Gene ID : 3606). La séquence de celui-ci est accessible sous l'une des références NCBI : NM_001562.3 et NM_001243211.1.

La métalloprotéinase MMP1 humaine possède une séquence protéique représentée par la séquence de référence GenBank: EAW67031.1 ou la séquence de référence NCBI NP_002412.1, codée par le gène MMP1 (référence NCBI : GeneID:4312).

Le facteur de nécrose tumorale alpha (TNFα) humain possède une séquence protéique représentée par exemple par la séquence de référence GenBank: QCI55793.1 ou GenBank: QCI55792.1, ou encore GenBank: QCI55791.1, codée par le gène TNF-alpha (référence NCBI : Gene ID : 7124).

Le marqueur de la vergeture αSMA (alpha-Smooth Muscle Actin) humain possède une séquence protéique représentée par exemple par la séquence de référence NP_001307784.1 ou la séquence de référence NP_001135417.1 ou la séquence de référence NP_001604.1. Cette protéine est codée par le gène *ACTA2* (référence NCBI : Gene ID : 59) dont la séquence correspond de préférence à la séquence de référence NM_001141945 ou à la séquence de référence NM_001613, ou encore à la séquence de référence NM_001320855.

Selon un mode de réalisation particulièrement préféré, l'utilisation de la présente composition cosmétique est une utilisation non thérapeutique. Selon un mode de réalisation particulièrement préféré, la présente méthode de soin cosmétique est une méthode non thérapeutique. Selon un mode de réalisation, la présente composition cosmétique n'a pas d'effet thérapeutique.

La présente invention concerne donc l'utilisation cosmétique (et/ou esthétique) et non thérapeutique d'une composition cosmétique telle que décrite ci-dessus, pour prévenir l'apparition des vergetures sur la peau et/ou réduire les vergetures sur la peau, en particulier pendant l'adolescence et/ou pendant la grossesse.

La présente invention concerne donc également une méthode de soin cosmétique et non thérapeutique de la peau, ainsi qu'une méthode de soin esthétique et non thérapeutique de la peau, pour prévenir l'apparition des vergetures sur la peau et/ou réduire les vergetures sur la peau, en particulier pendant l'adolescence et/ou pendant la grossesse, comprenant l'administration d'une composition cosmétique telle que décrite ci-dessus. Ainsi, l'invention concerne l'utilisation cosmétique (et/ou esthétique) et non thérapeutique, ainsi qu'une méthode de soin cosmétique (et/ou esthétique) et non thérapeutique, pour prévenir et/ou réduire les effets esthétiques et/ou cosmétiques indésirables des vergetures, tels que définis ci-dessus, par exemple tels que le changement de teinte et/ou de relief de la peau (notamment la rugosité, l'étirement et/ou la distension de la peau, la diminution ou la perte de fermeté et/ou la perte de l'élasticité, ainsi que les gênes, inconforts ou incommodités associés (démangeaisons, sensations de tiraillement, sensations d'inconfort).

Comme l'ont démontré les Inventeurs, la présente composition cosmétique est particulièrement adaptée aux femmes enceintes ou aux femmes ayant subi un accouchement. La présente utilisation cosmétique ou la présente méthode de soin cosmétique est donc avantageusement effectuée ou appliquée chez des femmes enceintes et/ou ayant accouché (telle que les femmes post-partum). Toutefois, les données obtenues à l'aide du modèle de vergetures de peau, développé par les inventeurs, montrent un effet anti-vergetures quelles que soient les conditions physiologiques, montrant ainsi que la composition cosmétique est adaptée à tout type de sujet et à tout type de peau. La présente composition peut être utilisée chez toute personne souhaitant prévenir et ou réduire les vergetures, notamment les adolescents, les femmes et les hommes ayant une tendance à développer des vergetures, les sujets dont au moins un des parents présente des vergetures, les sujets allant ou ayant subi une importante modification du poids corporel (perte ou gain, par exemple de masse musculaire et/ou graisseuse), les sportifs (les chocs pouvant favoriser les vergetures), etc.

Selon un mode de réalisation avantageux, la présente composition cosmétique est utilisée chez un sujet sain.

Dans l'utilisation cosmétique ou la méthode de soin cosmétique décrites ici, la présente composition cosmétique topique peut être appliquée sur la peau d'un sujet, de préférence un sujet sain, notamment sur les zones ayant une propension à développer les vergetures, telles que la face antérieure de l'abdomen, les seins, les cuisses et les hanches, ou encore les mollets. La composition cosmétique topique est de préférence appliquée sur la peau saine.

Dans l'utilisation cosmétique ou la méthode de soin cosmétique décrites ici, la composition cosmétique orale peut être administrée chez un sujet par voie orale, de préférence un sujet sain.

### Composition pharmaceutique pour son utilisation dans la prévention ou le traitement des vergetures de la peau

Selon un autre aspect, l'invention concerne une composition pharmaceutique telle que définie ci-dessus pour son utilisation dans la prévention ou le traitement des vergetures de la peau.

L'invention concerne également l'utilisation d'une composition pharmaceutique telle que définie ci-dessus pour la fabrication d'un médicament pour la prévention ou le traitement des vergetures de la peau.

L'invention concerne également une méthode de prévention ou de traitement des vergetures de la peau, comprenant l'administration d'une composition pharmaceutique telle que définie ci-dessus.

Avantageusement, la composition pharmaceutique comprend un excipient pharmaceutiquement acceptable, tel que décrit ci-dessus.

Selon un mode de réalisation, la composition pharmaceutique décrite plus haut est administrée chez des patients souffrant de syndromes ou maladies pouvant être associées à des vergetures, tels que l'obésité, la tuberculose, la fièvre typhoïde, le syndrome de Prader-Willi, le syndrome de Laurence-Moon Biedel et le syndrome d'Ehlers-Danlos, le syndrome de Cushing, le syndrome de Marfan, ou le syndrome de Beals (ou syndrome CCA (Contractures - arachnodactylie congénital)).

Les exemples qui suivent visent à illustrer la présente invention.

### DESCRIPTION DES FIGURES

Figure 1 : Immunomarquage de l'élastine (Flurophore AF488 (gris foncé, formes allongées)) et de la fibrilline-1 (Flurophore FITC (gris clair, formes allongées)) dans des biopsies de peau vergeturée ou non ; Noyaux contre-colorés à l'iodure de propidium (gris foncé, formes rondes diffuses).
Figure 2 : Quantification de l'élastine, de la fibronectine, du Collagène I et du collagène III produits par des fibroblastes de vergeture rouge (FVR) comparativement à des fibroblastes de peau saine (FS) - dosage ELISA dans les surnageants de culture
Figure 3 : Analyse de l'expression différentielle du transcriptome dans des fibroblastes de vergetures rouge (FVR) et des fibroblastes de peau saine (FS).
Figure 4 : Observation des fibres de collagène en lumière polarisée après coloration au Trichrome de Masson (la couleur gris clair représente l'orientation des fibres par rapport à l'axe de polarisation de la lumière).
Figure 5 : Immunomarquage du Collagène III ; Révélation peroxydase (gris foncé).
Figure 6 : Immunomarquage de l'élastine ; Flurophore AF488 (gris clair, formes allongées) ; Noyaux contre-colorés à l'iodure de propidium (gris foncé, formes rondes diffuses).
Figure 7 : Immunomarquage de la fibrilline-1 ; Flurophore FITC (gris clair, formes allongées) ; Noyaux contre-colorés à l'iodure de propidium (gris foncé, formes rondes diffuses).
Figure 8 : Immunomarquage de l'lnterleukine-1 alpha (IL1α) ; révélation à la peroxydase (gris foncé).
Figure 9 : Dosage de l'lnterleukine-6 dans les milieux de culture après 5 et 7 jours d'étirement.
Figure 10 : Dosage de l'lnterleukine-8 dans les milieux de culture après 3, 5 et 10 jours d'étirement.
Figure 11 : Dosage de MMP1 dans les milieux de culture après 3, 5, 7 et 10 jours d'étirement.

### EXEMPLES

### Exemple 1 : Etude de la physiologie des vergetures in vitro

La physiologie de la peau au niveau de vergetures a été étudiée. Le profil d'expression de marqueurs biologiques a été étudié dans des explants de peau ou dans des fibroblastes en culture provenant d'échantillons de peau avec vergetures (peau vergeturée) ou sans vergetures (peau normale).

1.1. Des explants de peau avec ou sans vergetures ont été fixées et des analyses histologiques ont été réalisées : coloration Verhoeff (marquage des fibres élastiques (élastine)); immunomarquages de la Fibrilline 1.

Coloration Verhoeff : Cette coloration met en évidence les fibres élastiques dans les tissus. Après déparaffinage, les coupes sont plongées dans les solutions suivantes : Verhoeff modifiée, chlorure ferrique et Van Gieson. Après déshydratation, les coupes sont montées entre lame et lamelle dans l'Entellan®. Les cellules sont de couleur brune. Les fibres élastiques apparaissent en bleu-marine/noir et les autres composants de la matrice extracellulaire en rouge.

Un marquage en immunofluorescence de la fibrilline 1 a été réalisé : Après fixation dans l'acétone, les coupes sont réhydratées et incubées avec l'anticorps primaire anti-Fibrilline 1. Après rinçage, les coupes sont incubées avec l'anticorps secondaire couplé à un marqueur fluorescent [Alexa Fluor® 488]. Les coupes sont montées entre lame et lamelle en milieu aqueux contenant du DAPI afin de contre-colorer l'ADN et mettre en évidence les noyaux.

La fibrilline 1 est une glycoprotéine, composante essentielle du réseau élastique du derme. Les fibres oxytalanes, allant jusqu'au contact de la jonction dermo-épidermique, sont des fibres très riches en fibrilline-1 recouvrant des fonctions essentielles dans le maintien de l'intégrité et des propriétés mécaniques de la peau.

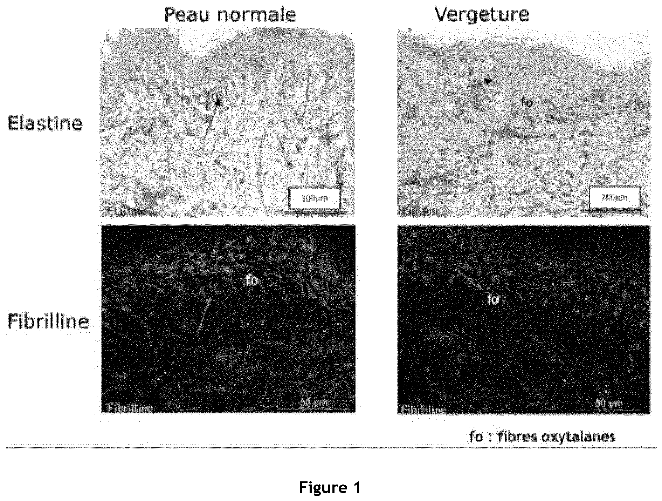

La figure 1 montre que le nombre et la taille de fibres élastiques et des fibres d'oxytalanes est diminuée dans la peau vergeturée, comparativement à la peau normale (peau non vergeturée).

1.2. Les fibroblastes sont isolés à partir d'échantillons de peau prélevée au niveau des vergetures de coloration rouge (vergetures rouges) chez 3 sujets et comparés aux fibroblastes isolés à partir de biopsies de peau prélevées au niveau de peau normale (sans vergetures) chez ces mêmes 3 sujets.

Les fibroblastes sont mis en culture, dans des conditions de culture classiques.

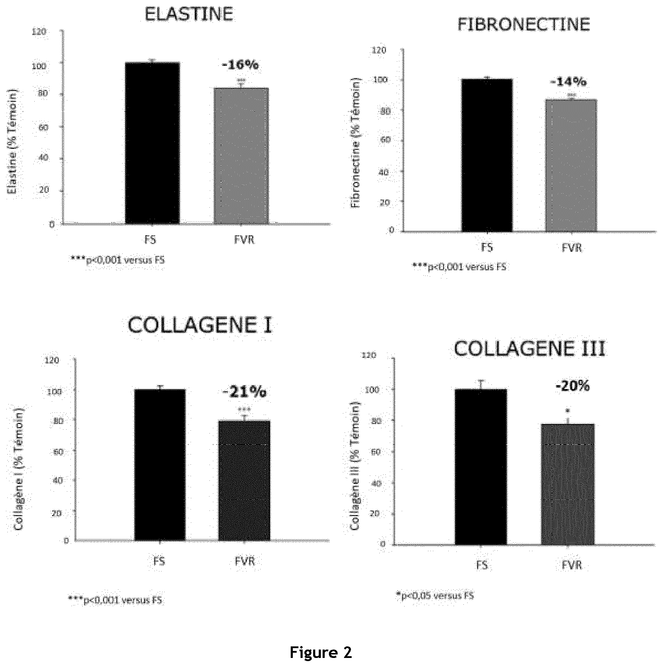

La production des marqueurs dermiques (marqueurs de l'organisation des fibres matricielles et particulièrement des fibres élastiques) que sont l'élastine, la fibronectine, le collagène I, et le collagène III (protéines matricielles) est analysée par mesure des niveaux protéiques (dosage ELISA). La Figure 2 montre que les niveaux d'expression de ces protéines est plus faible dans les fibroblastes de vergetures que dans les fibroblastes de la peau normale.

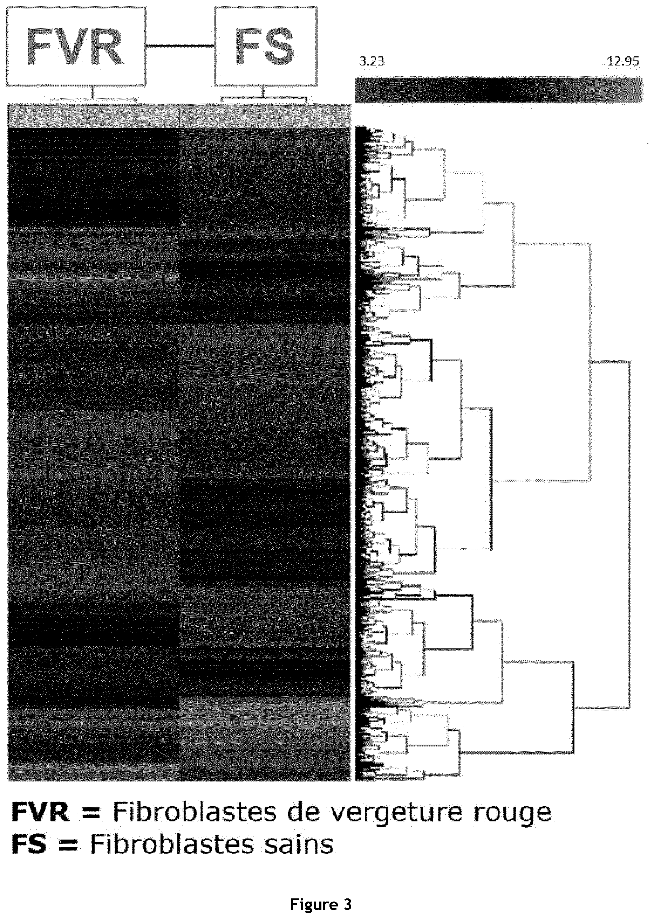

Une analyse transcriptomique est également réalisée, afin d'identifier les processus physiologies modulés dans les fibroblastes de vergetures, comparativement aux fibroblastes de peau normale (dits fibroblastes « sains »). Les résultats montrés sur la Figure 3 révèlent un profil d'expression très différents entre les fibroblastes de vergeture et des fibroblastes sains.

Une analyse par donneur est réalisée, les marqueurs pour lesquels les Fold Change (ou variations) étaient significativement régulés (≤x0,5 et ≥x2) pour au moins deux donneurs ont été retenus et sont récapitulés dans le tableau 4 ci-dessous.

Le tableau 4 ci-dessous montre les variations de niveaux d'expression de différents gènes dans les fibroblastes de vergeture comparativement aux fibroblastes sains, pour les 3 donneurs.

Les données montrent notamment que la production de TNFα est induite dans les fibroblastes de vergetures.

**Tableau 4 : Niveaux d'expression de différents gènes dans les fibroblastes de vergeture comparativement aux fibroblastes de peau normale, pour 3 donneurs.**

| | **Donneur 1** | **Donneur 2** | **Donneur 3** |
|---|---|---|---|
| **Angiopoietin like 4** | -- | -- | / |
| **Secretogranin II** | -- | -- | - |
| **Integrine 8** | -- | -- | - |
| **Endotheline-1** | -- | -- | ++ |
| **Protocadherine 10** | -- | -- | / |
| **Synémine** | -- | -- | / |
| **Zonula Occludens** | -- | -- | -- |
| **Dermokine** | -- | -- | -- |
| **Microfi brillar-associated protein 4** | -- | -- | -- |
| **Claudine 11** | -- | - | -- |
| **Syndécane 1** | ++ | ++ | -- |
| **Serpine 1** | | | |
| **Fibrilline 2** | ++ | ++ | - |
| **Plasminogen activator Tissue** | ++ | ++ | + |
| **Laminine 2** | ++ | ++ | - |
| **Prostaglandin E synthase** | ++ | ++ | - |
| **TIMP3** | ++ | - | ++ |
| **Spondine 1** | ++ | / | ++ |
| **Kératine7** | -- | ++ | ++ |
| **TNF** | ++ | ++ | ++ |

### Exemple 2 : Efficacité d'une Crème anti-Vergetures selon l'invention sur des explants de peau sous contrainte mécanique

L'étude consiste à placer des explants de peau sous contrainte mécanique afin de modéliser l'étirement de la peau, notamment au cours de la grossesse, dans le but d'étudier la formation des vergetures et d'évaluer l'efficacité d'un produit anti-vergetures ayant une composition selon la présente invention.

### 2.1. Matériel et Méthodes

Des explants de peau provenant d'une plastie abdominale d'une femme caucasienne de 43 ans ont été placés en survie pendant 10 jours entre deux mors, avec ou sans contrainte mécanique à force constante (une force constante uniaxiale de 1N, jusqu'à 50% d'allongement), à 37°C en atmosphère humide enrichie de 5% de CO2. L'application d'une telle tension mime donc l'étirement de la peau causant les vergetures, notamment au cours de la grossesse.

Les explants ont été traités, ou non, en surface par le produit « Crème anti-Vergetures » (ou « Crème Vergeture ») à raison de 2 mg/cm² à J0, J3, J5 et J7. Le milieu de culture a été renouvelé à J3, J5 et J7.

La composition de la crème anti-vergeture testée est indiquée dans le tableau 5.

**Tableau 5 : Composition de la Crème anti-Vergetures testée (les quantités sont indiquées en % en poids par rapport au poids total de crème).**

| *Ingrédient* | *% en poids* / *poids total de crème* |
|---|---|
| EAU PURIFIEE UP4 | 20-90% |
| DICAPRYLYL CARBONATE | 4-8% |
| CAPRYLATE/CAPRATE DE COPRAH | 4-8% |
| EMULIUM KAPPA2 | 2-8% |
| GLYCEROL | 0,5-10% |
| BEURRE DE KARITE | 0,5-10% |
| HYDROGENATED PALM GLYCERIDE | 0,5-5% |
| HYDROLITE-6 | 0,5-5% |
| HUILE AVOCAT | 0,5-10% |
| EXTRAIT D'HAMAMELIS | 0,0001-50% |
| POLYACRYLATE CROSSPOLYMER | 0,1-2% |
| ALPHA-TOCOPHEROL | 0.1-2% |
| OCTANEDIOL XI | 0.01-1,5% |
| PARFUM | 0.01-1,5% |
| EUMULGINS G | 0,001-1% |
| POLYPHENOLS DE MARACUJA | 0,0025-10% |
| PEPTIDES D'AVOCAT | 0.001-5% |
| ACIDE CITRIQUEI | 0,001-1% |
| LESSIVE SOUDE | 0,001-1% |
| TOTAL | 100 % |

A J3, J5, J7 et J10, du milieu de culture a été prélevé afin de procéder aux dosages ELISA des marqueurs suivants : Interleukine 8 (IL8), Interleukine 6 (IL6), Matrix-Metalo-Proteinase-1 ou métalloprotéinase 1 (MMP1).

Des analyses histologiques ont été réalisées à J0 (pour la condition contrôle) et à J10 (pour l'ensemble des conditions) : coloration au trichrome de Masson ; immunomarquages de l'lnterleukine-1 alpha (IL1α), Elastine, Fibrilline 1, Collagène III.

Coloration au trichrome de Masson : L'orientation des faisceaux de collagène a été observée en lumière polarisée sur les coupes histologiques après coloration au trichrome de Masson.

Immunomarquage de l'interleukine-1 alpha : L'interleukine-1 alpha a été marquée sur coupes en paraffine formol avec un anticorps monoclonal anti- IL-1 alpha, pendant 1h à température ambiante, avec un système amplificateur biotine/ streptavidine et révélé en VIP, un substrat violet de peroxydase.

Immunomarquage de l'Elastine : L'élastine a été marquée sur coupes congelées avec un anticorps polyclonal anti-élastine pendant 1h à température ambiante et révélé par le fluorophore AF488. Les noyaux ont été contre-colorés à l'iodure de propidium. Immunomarquage de la Fibrilline-1 : la fibrilline-1 a été marquée sur coupes congelées avec un anticorps monoclonal anti-fibrilline 1 pendant 1 h à température ambiante, avec un système amplificateur biotine/streptavidine et révélé en FITC. Les noyaux ont été contre-colorés à l'iodure de propidium.

Immunomarquage du Collagène III : le collagène III a été marqué sur coupes congelées avec un anticorps polyclonal anti-collagène III pendant 2 heures à température ambiante, avec un système amplificateur biotine/streptavidine et révélé en VIP, un substrat violet de la peroxydase.

Les conditions à l'essai sont les suivantes :
- Contrôle : Explants à J0
- Témoin non étiré : Explants non traités, non étirés (non soumis à la contrainte mécanique), à J10
- Témoin étiré : Explants non traités, étirés (soumis à la contrainte mécanique), à J10
- Non étiré + Produit : Explants traités par la crème anti-vergeture, non étirés, à J10
- Etiré + Produit : Explants traités, étirés, à J10.

### 2.2. Résultats

### 2.2.1. Observation des fibres de Collagène

L'observation microscopique en lumière polarisée des coupes après coloration au trichrome de Masson permet de visualiser l'orientation des fibres de collagène.

Sous lumière polarisée, les couleurs observées dépendent de l'orientation de la fibre. Une lumière polarisée (filtre polariseur) est envoyée sur l'échantillon qui en fonction de l'orientation des fibres de collagène modifie la polarisation de la lumière. Un filtre dit analyseur permet de sélectionner la lumière renvoyée. En réglant les deux filtres on obtient ces différentes couleurs du bleu au rose (du gris + ou - clair sur les figures) en fonction de l'orientation de la fibre par rapport à l'axe de polarisation de la lumière.

Si toutes les fibres sont dans le même axe, il y a une prédominance d'une seule couleur. La coloration trichrome de Masson est utilisée pour différencier les fibres de collagène et le tissu musculaire dans des coupes histologiques. Comme son nom l'indique, cette coloration associe trois colorants :
- un colorant nucléaire violet (hématoxyline),
- un colorant cytoplasmique rouge (fuchsine ponceau), et
- un colorant des fibres de collagène vert (vert lumière).

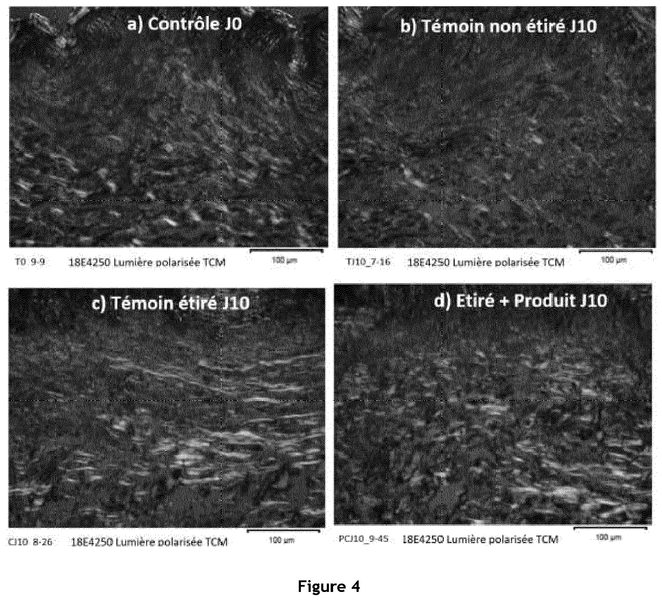

Les explants non étirés, à J0 et à J10, ne présentent pas d'orientation prédominante des fibres de collagène (figures 4a et 4b). Dans les explants soumis à la contrainte mécanique, les fibres de collagène sont très nettement orientées dans l'axe de la tension (figure 4c). Dans ces conditions, l'application de la crème anti-vergetures a permis de préserver l'orientation des fibres induite par l'étirement (figure 4d). En effet, celles-ci ne suivent pas une orientation prédominante.

### 2.2.2. Expression du Collagène III dans les explants

Le collagène de type III est un collagène fibrillaire qui s'associe de façon très organisée avec les collagènes de type I et V pour former les fibres de collagène. Le collagène III représente 10 à 15% des fibres de collagène du derme, il est principalement impliqué dans la réticulation des fibres de collagène.

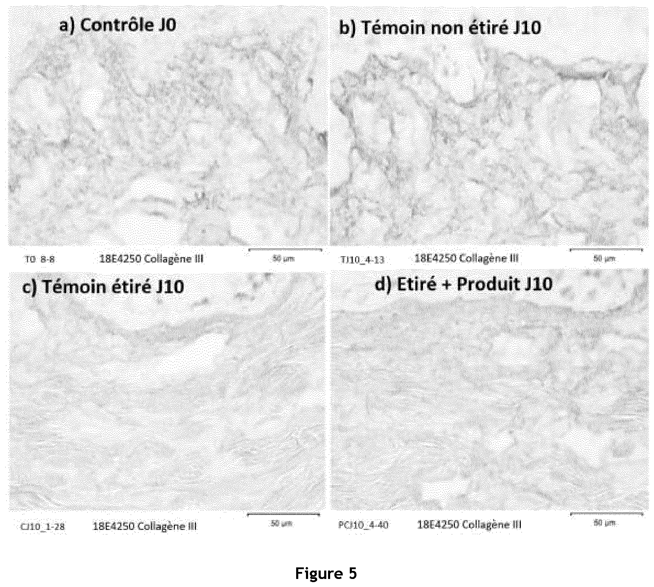

Dans les conditions de mise sous contrainte mécanique des explants (figure 5c), le marquage du collagène III est diminué, comme dans la peau vergeturée, in vivo (voir l'exemple 1 ci-dessus). Ainsi, le modèle de peau reproduit fidèlement les caractéristiques de la vergeture.

Ce marquage présente également une orientation des fibres dans le sens de l'étirement. L'application du produit dans les conditions d'étirement (figure 5d) entraine une augmentation du niveau d'expression du Collagène III. Ce résultat montre que la crème anti-vergetures a un effet protecteur du réseau de collagène.

### 2.2.3. Expression de l'Elastine dans les explants

L'élastine est une protéine du tissu conjonctif responsable des propriétés élastiques de la peau ; elle est la composante majeure du réseau élastique de la peau.

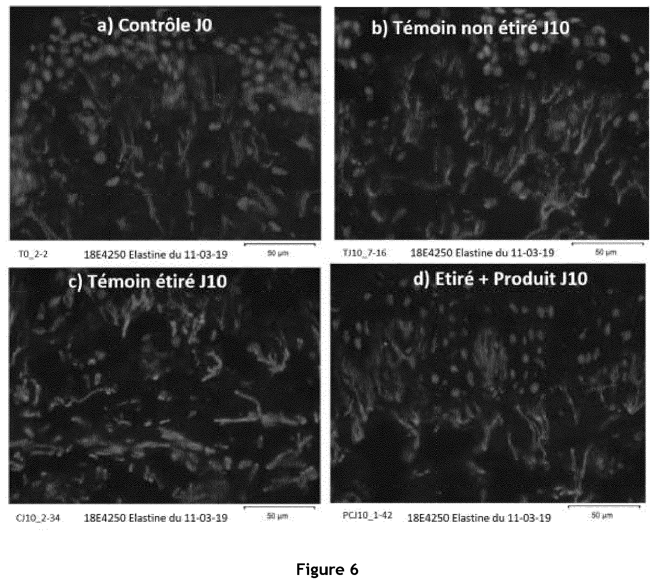

Dans les conditions contrôles à J0 (figure 6a) et J10 (figure 6b), le marquage de l'élastine est assez net dans le derme papillaire. En condition étirée (figure 6c), le niveau d'expression de l'élastine est peu modifié. En revanche, l'organisation du réseau de fibres élastiques est modifiée : raccourcissement des fibres et orientation dans le sens de l'étirement, comme dans la peau vergeturée, in vivo (voir l'exemple 1 ci-dessus). Ainsi, le modèle de peau reproduit fidèlement les caractéristiques des fibres d'élastine de la vergeture.

Dans ces conditions d'étirement, la crème anti-vergetures permet de préserver l'organisation et l'orientation des fibres élastiques (figure 6d).

### 2.2.4. Expression de la Fibrilline 1 dans les explants

La fibrilline 1 est une glycoprotéine, composante essentielle du réseau élastique du derme. Les fibres oxytalanes, allant jusqu'au contact de la jonction dermo-épidermique, sont des fibres très riches en fibrilline-1 recouvrant des fonctions essentielles dans le maintien de l'intégrité et des propriétés mécaniques de la peau.

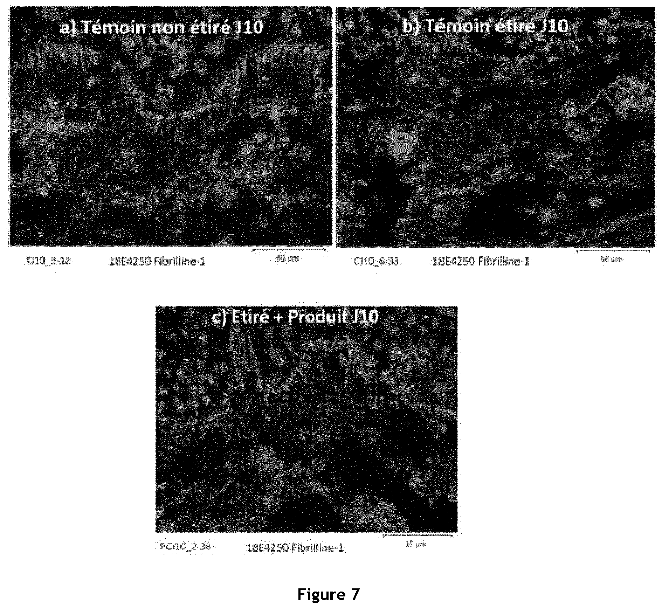

La mise sous contrainte mécanique des explants a entrainé une modification du réseau de fibres oxytalanes riches en fibrilline-1. En effet, dans les conditions d'étirement (figure 7b), le réseau de fibres oxytalanes est altéré : le réseau est très fin sous la jonction dermo-épidermique et les fibres raccourcies, comme dans la peau vergeturée, in vivo (voir l'exemple 1 ci-dessus). Ainsi, le modèle de peau reproduit fidèlement les caractéristiques des fibres oxytalanes de la vergeture. Cette altération est peu ou pas observée en présence de la crème anti-vergetures (figure 7c), ce qui montre que l'application de la crème a un effet protecteur du réseau de fibres oxytalanes.

### 2.2.5. Expression d'lL1a dans les explants

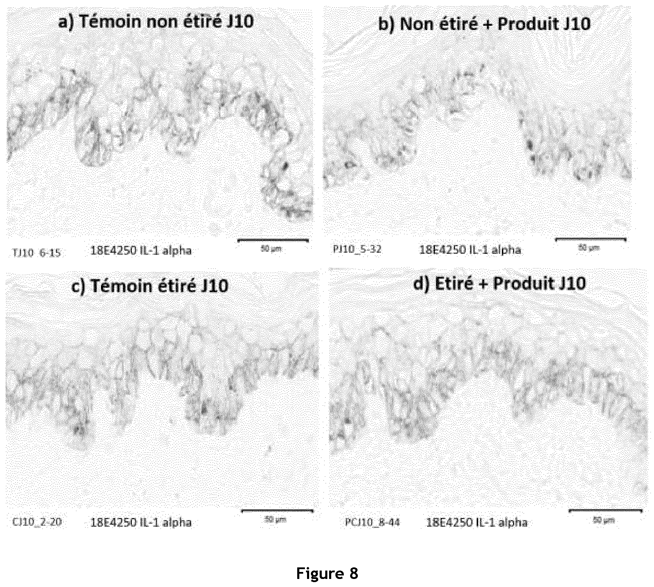

L'application de la crème anti-vergetures sur les explants a induit une nette diminution du marquage de l'IL1α, que ce soit en condition étirée (Figure 8d) ou non étirée (figure 8b).

Ces résultats suggèrent que la crème anti-vergeture exerce une activité anti-inflammatoire.

### 2.2.6. Production d'IL6

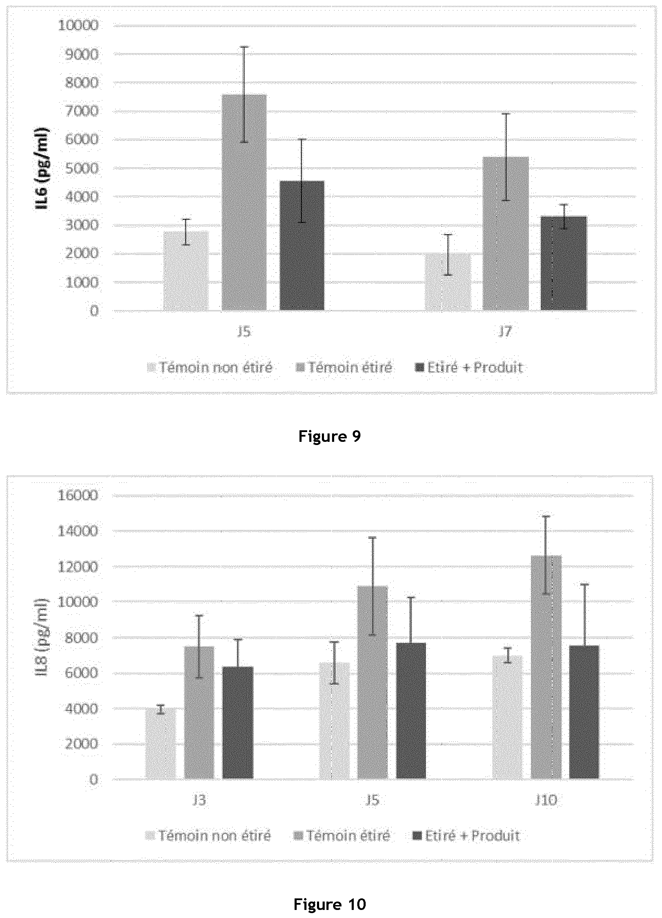

L'étirement des explants a induit une augmentation du relargage d'IL6. Ainsi, l'étirement stimule un signal pro-inflammatoire propice au développement des vergetures. L'application du produit anti-vergetures permet d'inhiber ce relargage. Ce résultat suggère une activité anti-inflammatoire de la crème anti-vergetures (Tableau 6 et Figure 9).

**Tableau 6 : Dosage de l'lnterleukine-6 dans les milieux de culture après 5 jours et 7 jours d'étirement - Quantité en pg/ml - *p<0,05, ns Non significatif, test t de Student**

| | | IL6 (pg/ml) | Evolution |
|---|---|---|---|
| J5 | Témoin non étiré J5 | 2764 ± 456 | |
| | Témoin étiré J5 | 7592 ± 1663 | +175% * (vs Témoin non étiré) |
| | Etiré + Produit J5 | 4554 ± 1458 | -40% ns (vs Témoin étiré) |
| J7 | Témoin non étiré J7 | 1966 ± 711 | |
| | Témoin étiré J7 | 5395 ± 1522 | +174% * (vs Témoin non étiré) |
| | Etiré + Produit J7 | 3306 ± 411 | -39% ns (vs Témoin étiré) |

### 2.2.7. Production d'IL8

L'étirement des explants a induit une augmentation du relargage d'IL8 ; l'application du produit anti-vergetures tend à inhiber ce relargage, suggérant une activité anti-inflammatoire (Tableau 7 et Figure 10).

**Tableau 7: Dosage de l'lnterleukine-8 dans les milieux de culture après 3, 5 et 10 jours d'étirement - Quantité en pg/ml - *p<0,05, ns Non significatif, test t de Student**

| | | IL8 (pg/ml) | Evolution |
|---|---|---|---|
| J3 | Témoin non étiré J3 | 3967 ± 239 | |
| | Témoin étiré J3 | 7507 ± 1760 | +89% * (vs Témoin non étiré) |
| | Etiré + Produit J3 | 6372 ± 1542 | -15% ns (vs Témoin étiré) |
| J5 | Témoin non étiré J5 | 6580 ± 1184 | |
| | Témoin étiré J5 | 10884 ± 2734 | +65% ns (vs Témoin non étiré) |
| | Etiré + Produit J5 | 7701 ± 2585 | -29% ns (vs Témoin étiré) |
| J10 | Témoin non étiré J10 | 6999 ± 409 | |
| | Témoin étiré J10 | 12639 ± 2200 | +81% * (vs Témoin non étiré) |
| | Etiré + Produit J10 | 7554 ± 3423 | -40% ns (vs Témoin étiré) |

### 2.2.8. Production de MMP1

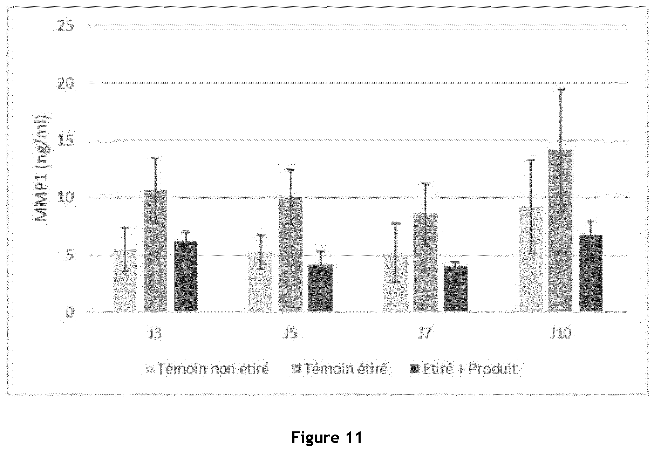

L'étirement des explants a induit une stimulation du relargage de MMP1, cette métalloprotéinases, induite en conditions inflammatoires, est responsable de la dégradation des fibres matricielles, en particulier des fibres de collagène. L'application du produit anti-vergetures a inhibé le relargage de la MMP1. Ce résultat montre que la crème a un effet anti-inflammatoire et protecteur de la matrice dermique (Tableau 8 et Figure 11).

**Tableau 8: Dosage de la MMP1 dans les milieux de culture après 3, 5, 7 et 10 jours d'étirement - Quantité en ng/ml - *p<0,05, ls Limite de significativité, ns Non significatif, test t de Student**

| | | MMP1 (ng/ml) | Evolution |
|---|---|---|---|
| J3 | Témoin non étiré | 5.484 ± 1.913 | |
| | Témoin étiré | 10.605 ± 2.871 | +93% ns (vs Témoin non étiré) |
| | Etiré + Produit | 6.161 ± 0.807 | -42% ns (vs Témoin étiré) |
| J5 | Témoin non étiré | 5.269 ± 4.489 | |
| | Témoin étiré | 10.084 ± 2.339 | +91% ls (vs Témoin non étiré) |
| | Etiré + Produit | 4.163 ± 1.200 | -59% * (vs Témoin étiré) |
| J7 | Témoin non étiré | 5.185 ± 2.568 | |
| | Témoin étiré | 8.584 ± 2.641 | +66% ns (vs Témoin non étiré) |
| | Etiré + Produit | 4.101 ± 0.258 | -52% ls (vs Témoin étiré) |
| J10 | Témoin non étiré | 9.205 ± 3.996 | |
| | Témoin étiré | 14.104 ± 5.358 | +53% ns (vs Témoin non étiré) |
| | Etiré + Produit | 6.792 ± 1.122 | -52% ns (vs Témoin étiré) |

### 2.3. Conclusion

La contrainte mécanique à force constante appliquée pendant 10 jours a induit des modifications de la matrice extra cellulaire dermique similaires à ce qui est observé dans des zones de vergetures in vivo : orientation des fibres de collagène et d'élastine dans le sens de la tension ; altération des fibres oxytalanes sous la jonction dermo-épidermique ; diminution de l'expression du collagène III au niveau des fibres réorientées ; induction d'une inflammation via le relargage de cytokines (IL6, IL8, IL1α).

Les données montrent que la crème anti-vergetures présente une activité protectrice sur ces différents paramètres.

L'application de la crème anti-vergetures induit notamment :
- Préservation de l'organisation des fibres de collagène et d'élastine par le produit dans les conditions d'étirement (analyses histologiques : morphologie globale et immuno-marquages) ;
- Inhibition des médiateurs inflammatoires dans les conditions de l'étirement.

Ainsi dans un modèle in vitro reproduisant l'étirement de la peau au cours de la grossesse conduisant à la formation de vergetures, le produit anti-vergetures évalué préserve les structures matricielles du derme. Cette crème permet donc de préserver les propriétés mécaniques de la peau soumise à un étirement mécanique.

Par ailleurs, par ces propriétés anti-inflammatoires, le produit permet de moduler l'inflammation observée au cours de la formation des vergetures et impliquée dans la physiologie de celle-ci.

### Exemple 3 : Evaluation de l'effet hydratant d'une Crème anti-Vergetures selon l'invention sur la peau des femmes

Le niveau d'hydratation de la peau a une incidence sur l'apparition des vergetures. Il est donc avantageux qu'une crème anti-vergetures ait en outre un effet hydratant.

Cette étude consiste en un test d'objectivation clinique réalisé en Pologne pour évaluer l'effet du produit (la crème anti-vergetures dont la composition est présentée dans le tableau 5 ci-dessus) sur la perte insensible en eau et son effet hydratant. L'effet est évalué 1h, 4h, 8h et 24h après application unique standardisée de cette crème, auprès de 22 femmes âgées de 24 à 65 ans, présentant une peau de nature sèche au niveau des avant-bras (taux d'hydratation cutané ≤50 u.a.).

### 3.1. Déroulement de l'étude :

- Définition de 2 zones au niveau des avant-bras pour les mesures (une zone traitée par la crème anti-vergetures selon le tableau 5) et une zone témoin non traitée) ;
- Mesure de la perte insensible en eau (P.I.E.) à l'aide d'un Tewamètre et du taux d'hydratation à l'aide d'un cornéomètre à J0 T0 au niveau des 2 zones précédemment définies ;
- Application unique standardisée par le technicien de 2 µL/cm² du produit à tester (la crème anti-vergetures selon le tableau 5 ci-dessus) sur la zone traitée précédemment définie ;
- A t1h, t4h, t8h et t24h après application du produit, nouvelles mesures de la perte insensible en eau (P.I.E.) à l'aide du Tewamètre et du taux d'hydratation à l'aide du cornéomètre au niveau des 2 zones précédemment définies.

### 3.2. Résultats :

### 3.2.1. Effet sur la Perte Insensible en Eau (P.I.E.) :

Le Tableau 9 montre une diminution statistiquement significative de la P.I.E. dans le temps tenant compte des variations physiologiques de la zone témoin à t4h, t8h et t24h. En revanche, pas d'évolution statistiquement significative à t1h.

**Tableau 9: Effet sur la Perte Insensible en Eau (P.I.E.). S = significatif.**

| | | |
|---|---|---|
| PIE au temps 4 heures (n=22) | *-6%* | *S* |
| PIE au temps 8 heures (n=20) | -*5%* | *S* |
| PIE au temps 24 heures (n=20) | *-4%* | *S* |

### 3.2.2. Effet sur le taux d'hydratation des couches superficielles de l'épiderme :

Le Tableau 10 montre une augmentation statistiquement significative du taux d'hydratation cutané dans le temps tenant compte des variations physiologiques de la zone témoin, à t1h, t4h et t8h. En revanche, pas d'évolution statistiquement significative à t24h.

**Tableau 10: Effet sur le taux d'hydratation des couches superficielles de l'épiderme. S = significatif.**

| | | |
|---|---|---|
| Gain d'hydratation au temps 1 heure (n=22) | +*42%* | *S* |
| Gain d'hydratation au temps 4 heures (n=22) | +*31%* | *S* |
| Gain d'hydratation au temps 8 heures (n=21) | +*10%* | *S* |

### 3.3. Conclusion :

Les données montrent :
- Effet protecteur de la crème anti-vergetures et amélioration de la barrière cutanée à t4h et jusqu'à t24h
- La crème respecte et préserve la barrière cutanée jusqu'à t24h
- Effet hydratant longue durée de la crème jusqu'à t8h.

### Exemple 4 : Efficacité d'une Crème anti-Vergetures selon l'invention sur la prévention et la réduction des vergetures sur la peau chez des femmes enceintes

Cette étude visait à d'évaluer l'efficacité d'une crème anti-vergetures selon l'invention (crème ayant une composition selon le tableau 5 ci-dessus), sur la prévention et la réduction des vergetures sur la peau chez des femmes enceintes. Cette étude a été réalisée en Pologne, en ouvert avec 1 médecin dermatologue et 4 médecins gynécologues auprès de 64 femmes enceintes du 4^{ème} mois de grossesse à 6 semaines après l'accouchement, âgées de 20 à 35 ans. L'ensemble des sujets a été examiné par le dermatologue et un médecin gynécologue.

**Tableau 11: Sujets inclus dans l'étude.**

| | |
|---|---|
| TOTAL des sujets inclus | 66 |
| Perdus de vue | 0 |
| Dossiers exclus | 0 |
| Arrêts prématurés sans effet indésirable | 0 |
| Arrêts prématurés avec effet indésirable | 2 |
| TOTAL des sujets pour l'analyse des résultats | 64 |

### 4.1. Modalités d'utilisation :

Utiliser 2 fois par jour sur l'ensemble du corps pendant 4 semaines dans les conditions normales d'utilisation.

### 4.2. Déroulement de l'étude :

**Tableau 12: Déroulement de l'étude.**

| | | |
|---|---|---|
| J0 | : | inclusion des sujets et examens cliniques réalisés par 1 dermatologue et 1 gynécologue |
| | | remise du produit testé |
| J84±10J | : | examens cliniques réalisés par 1 dermatologue et 1 gynécologue auto-évaluation et appréciation globale de l'utilisateur et de l'examinateur (dermatologue) |
| | | remontée de tolérance quotidienne par l'utilisateur |
| W6±10J | : | examens cliniques réalisés par 1 dermatologue et 1 gynécologue auto-évaluation et appréciation globale de l'utilisateur et de l'examinateur (dermatologue) |
| | | remontée de tolérance quotidienne par l'utilisateur |

### 4.3. Résultats :

### 4.3.1. EVALUATION DE LA TOLERANCE (n=66)

L'application répétée du produit testé (crème anti-vergetures selon le tableau 5) dans des conditions normales d'utilisation n'induit pas de réactions cutanées. Le produit est donc très bien toléré sur le plan cutané.

### 4.3.3. EVALUATION DE L'EFFICACITE (n=64)

Le tableau 13 ci-dessous présente les résultats d'une évaluation par l'examinateur (un dermatologue et un gynécologue) et indique le pourcentage d'avis favorable pour chaque critère à évaluer.

**Tableau 13: Appréciation globale de l'examinateur.**

| | *D84 (n=64)* | | *W6 (n=64)* | |
|---|---|---|---|---|
| | *% d'avis favorable* | *Significativité* | *% d'avis favorable* | *Significativité* |
| Le produit a une efficacité hydratante | *100%* | *S* | *100%* | *S* |
| Le produit a une efficacité adoucissante | *100%* | *S* | *100%* | *S* |
| Le produit a une efficacité nourrissante | *100%* | *S* | *100%* | *S* |
| Le produit a une efficacité préventive sur l'apparition des vergetures | *92%* | *S* | *80%* | *S* |
| Le produit permet à la peau de mieux résister aux étirements de la grossesse | *95%* | *S* | *81%* | *S* |
| Globalement, le produit est satisfaisant | *95%* | *S* | *86%* | *S* |

### S : significatif (pourcentage de réponses favorables ≥ 60.9%)

Les tableaux 14 à 16 ci-dessous présentent les résultats d'une auto-évaluation par les sujets inclus dans l'étude et indique le pourcentage d'avis favorable pour chaque critère à évaluer.

**Tableau 14: Auto-évaluation de l efficacité anti-vergetures de la crème par l'utilisatrice.**

| | *D84 (n=64)* | | *W6 (n=64)* | |
|---|---|---|---|---|
| Efficacité | *% d'avis favorable* | *Significativité* | *% d'avis favorable* | *Significativité* |
| Le produit rend la peau plus douce | *94%* | *S* | *100%* | *S* |
| Le produit rend la peau plus souple | *92%* | *S* | *89%* | *S* |
| Le produit rend la peau plus confortable | *94%* | *S* | *96%* | *S* |
| Le produit renforce l'élasticité de la peau | *98%* | *S* | *91%* | *S* |
| Le produit a une efficacité hydratante | *100%* | *S* | *100%* | *S* |
| Le produit a une efficacité nourrissante | *87%* | *S* | *94%* | *S* |
| Le produit a une efficacité apaisante | *75%* | *S* | *83%* | *S* |
| Le produit améliore l'aspect de la peau | *87%* | *S* | *94%* | *S* |
| Le produit a une efficacité préventive sur l'apparition des vergetures | *84%* | *S* | *77%* | *S* |
| Le produit permet à la peau de mieux résister aux étirements de la grossesse | *92%* | *S* | *84%* | *S* |
| Le produit diminue immédiatement les sensations de tiraillement liées à la grossesse | *81%* | *S* | *84%* | *S* |
| Le produit diminue durablement les sensations de tiraillement liées à la grossesse | *75%* | *S* | *83%* | *S* |
| Le produit diminue immédiatement les démangeaisons liées à la grossesse | *72%* | *S* | *77%* | *S* |
| Le produit diminue durablement les démangeaisons liées à la grossesse | *63%* | *S* | *76%* | *S* |
| Le produit a une action sur les vergetures naissantes (panel concerné : n=11 sujets à D84 et n=26 sujets à W6) | *54%* | *NS* | *38%* | *NS* |
| Le produit aide à maintenir le bon état de la peau | *98%* | *S* | *95%* | *S* |
| Le produit aide à limiter l'apparition des vergetures | *87%* | *S* | *86%* | *S* |
| Ma peau de femme enceinte tolère parfaitement le produit | *98%* | *S* | *100%* | *S* |

| | | | | |
|---|---|---|---|---|
| *S* : *significatif (pourcentage de réponses favorables ≥ 60.9%)* | | | | |

**Tableau 15: Auto-évaluation des propriétés cosmétiques de la crème anti-vergetures par l'utilisatrice.**

| | *D84 (n=64)* | | *W6 (n=64)* | |
|---|---|---|---|---|
| Propriétés cosmétique | *% d'avis favorable* | *Significativité* | *% d'avis favorable* | *Significativité* |
| La texture du produit est onctueuse | *99%* | *S* | *100%* | *S* |
| La texture du produit est ultra-nourrissante | *92%* | *S* | *92%* | *S* |
| La texture est riche | *84%* | *S* | *90%* | *S* |
| La texture du produit est agréable | *98%* | *S* | *99%* | *S* |
| La texture est non grasse | *89%* | *S* | *89%* | *S* |
| La consistance du produit est adaptée au massage | *86%* | *S* | *86%* | *S* |
| L'odeur du produit est agréable | *81%* | *S* | *87%* | *S* |
| L'odeur du produit est délicate | *89%* | *S* | *89%* | *S* |
| La senteur est adaptée à l'odorat sensible de la femme enceinte | *87%* | *S* | *84%* | *S* |
| Le produit n'irrite pas la peau | *95%* | *S* | *98%* | *S* |

| | | | | |
|---|---|---|---|---|
| *S : significatif (pourcentage de réponses favorables ≥ 60.9%)* | | | | |

**Tableau 16: Auto-évaluation de l'agréabilité de la crème anti-vergetures par l'utilisatrice.**

| | *D84 (n=64)* | | *W6 (n=64)* | |
|---|---|---|---|---|
| Agrément d'utilisation | *% d'avis favorable* | *Significativité* | *% d'avis favorable* | *Significativité* |
| Le produit est facile à appliquer | *100%* | *S* | *100%* | *S* |
| Le produit pénètre rapidement | *87%* | *S* | *91%* | *S* |
| La peau n'est pas collante après l'application | *91%* | *S* | *89%* | *S* |
| Le produit apporte un confort cutané immédiat | *97%* | *S* | *92%* | *S* |
| Le produit apporte un confort cutané durable | *94%* | *S* | *95%* | *S* |
| Le produit procure une sensation de bien-être | *89%* | *S* | *96%* | *S* |
| L'odeur du produit ne semble pas déranger bébé au moment de la tétée | *NA* | | *91%* | *S* |
| Après l'application du produit, il est possible de se rhabiller immédiatement | *69%* | *S* | *75%* | *S* |
| Le produit ne tache pas les vêtements | *92%* | *S* | *94%* | *S* |
| C'est un produit que vous recommanderiez à votre entourage | *98%* | *S* | *92%* | *S* |
| C'est un produit que vous achèteriez volontiers | *94%* | *S* | *89%* | *S* |
| Globalement, le produit est satisfaisant | *99%* | *S* | *92%* | *S* |

| | | | | |
|---|---|---|---|---|
| *S : significatif (pourcentage de réponses favorables ≥ 60.9%)* | | | | |

L'efficacité globale de la crème anti-vergetures a été évaluée en déterminant le pourcentage de sujets n'ayant pas développé de nouvelles vergetures, après 84 jours et 6 semaines d'utilisation.

Le tableau 17 ci-dessous montre les résultats, exprimé en considérant toutes les zones de manière globale (Panel global) et par zone (ventre, hanches, cuisses, fesses et poitrine).

**Tableau 17: Pourcentage de sujets n'ayant pas développé de nouvelles vergetures (auto-évaluation par l'utilisatrice).**

| | Panel Global | Ventre | Hanches | Cuisses | Fesses | Poitrine |
|---|---|---|---|---|---|---|
| J84 (n=65) | 83% | 97% | 98% | 98% | 98% | 89% |
| W6 (n=64) | 59% | 73% | 91% | 89% | 94% | 80% |

### 4.4. Conclusion

La crème anti-vergeture présente une tolérance et une efficacité significativement satisfaisantes. Les données cliniques montrent que son utilisation régulière permet de prévenir la formation de nouvelles vergetures et de réduire les vergetures déjà présentes, chez des femmes enceintes à différents stades de la grossesse ou après l'accouchement.

### Exemple 5 : Supériorité de l'efficacité d'une Composition anti-Vergetures selon l'invention sur des modèles d'étirement de cellules en monocouches

L'objectif de l'étude était de soumettre des fibroblastes et des kératinocytes à un étirement mécanique représentatif de l'étirement subi par la peau au cours de la grossesse et pouvant conduire à l'apparition des vergetures. La production de médiateurs inflammatoires en réponse à l'étirement a été étudiée, en présence ou en l'absence d'actifs.

### 5.1. Matériels & Méthodes

Des kératinocytes épidermiques humains normaux (KHN), d'une part, et des fibroblastes dermiques humains normaux (FHN), d'autre part, ont été ensemencés dans le système Flexercell®.

Le Flexercell® FX4000™ (Dunn Laborteknik, Allemagne) permet de soumettre des cellules adhérentes à une tension mécanique : les cellules sont ensemencées à la surface d'un support de culture flexible (Uniflex™ culture plate, Dunn Laborteknik) sur lequel une tension mécanique uniaxiale est appliquée.

Les cellules ont été soumises à un protocole d'étirement progressif pendant 3 jours, à une fréquence de 0,5Hz. Avec application d'une déformation de 10% pendant 24h le 1^{er} jour, 20% pendant 24h le 2^{ème} jour et 30% pendant 24h le 3^{ème} jour. Ce protocole d'étirement progressif a été mis en œuvre afin de modéliser l'étirement progressif de la peau au cours de la grossesse.

Les actifs ci-dessous seuls ou en combinaison ont été appliqués, ou non, dans les milieux de culture pendant toute la durée de l'étirement :
- Extrait d'hamamélis (A1) à 0,1%,
- Extrait de polyphénols de graines de passiflore (A2) à 0,02%,
- Extrait de peptides d'avocat (A3) à 0,01%,
- Combinaison des 3 actifs A1 0,1% + A2 0,02% + A3 0,01%.

A l'issue des 3 jours de traitement, la production et l'expression de médiateurs inflammatoires ont été évalués par dosage ELISA dans les milieux de culture ou par RT-PCR en temps réel dans les extraits cellulaires.

### 5.2. Résultats

L'étirement progressif appliqué sur les tapis cellulaires a significativement induit la production de médiateurs de l'inflammation par les kératinocytes (MMP1 et TNFα, tableau 18).

Ces résultats confirment l'impact de l'étirement sur la signalisation inflammatoire ainsi que les désordres matriciels observés dans le cadre de la physiophathologie des vergetures.

Dans ces conditions, les actifs appliqués seuls ou en combinaison ont significativement inhibé la production de ces médiateurs inflammatoires.

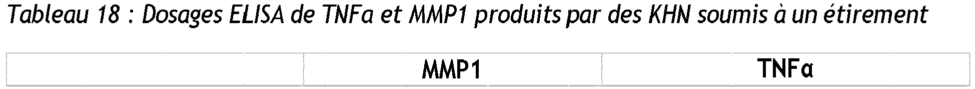

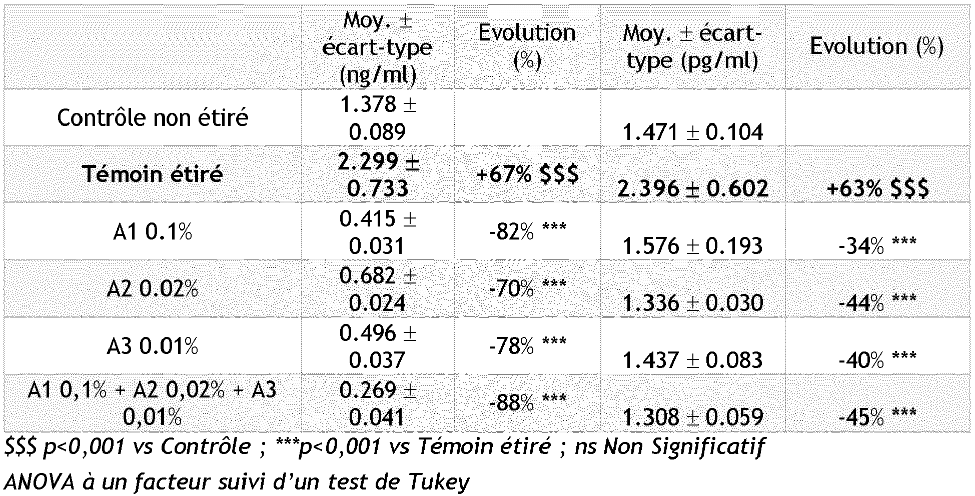

Dans les fibroblastes, l'étirement mécanique a significativement induit l'expression génique de αSMA (alpha-Smooth Muscle Actin) (tableau 19).

L'αSMA est un marqueur de la vergeture, en effet il a été décrit qu'au cours des premiers stades de formation de la vergeture (vergeture rouge), les fibroblastes répondent aux tensions mécaniques en exprimant l'αSMA et en produisant de puissantes forces contractiles (Viennet C. et al. (2005) Contractile Forces Generated by Striae Distensae Fibroblasts Embedded in Collagen Lattices. Archives of Dermatological Research, 297, 10-17).

Ainsi, l'augmentation de l'expression de αSMA confirme que ce modèle mime la formation de vergeture de manière fidèle.

Les actifs appliqués seuls ou en combinaison ont modulé la surexpression de l'αSMA (Tableau 19).

Il est à noter que l'effet des 3 actifs appliqués en combinaison est significativement plus important que les actifs appliqués séparément sur la modulation d'expression de l'αSMA.

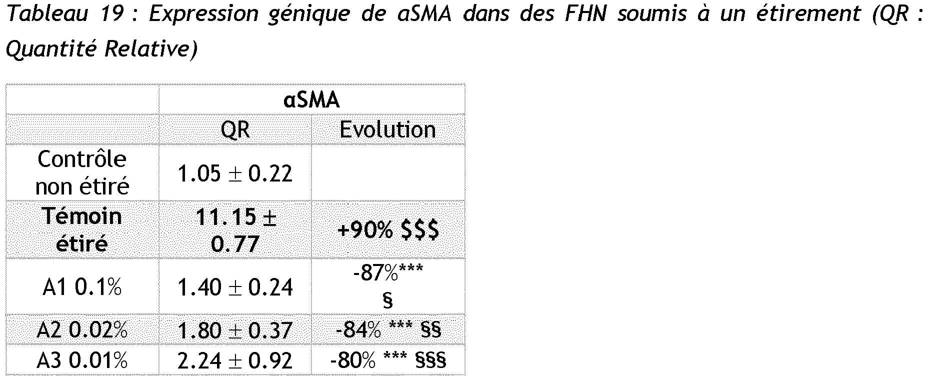

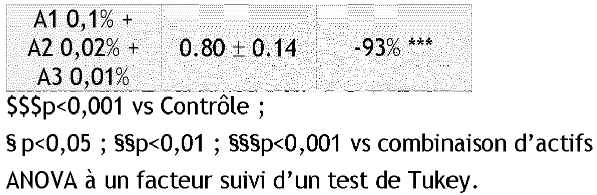

## Revendications

1. Composition comprenant :
- un extrait de polyphénols de graines de passiflore ;
- un extrait de peptides d'avocat ; et
- un extrait d'hamamélis.

2. Composition selon la revendication 1, dans laquelle la teneur en polyphénols de graines de passiflore va de 0,0025% à 10% en poids par rapport au poids total de la composition ; et dans laquelle les graines de passiflore sont notamment de graines de *Passiflora incarnata* ou de *Passiflora edulis.*

3. Composition selon la revendication 1 ou 2, dans laquelle la teneur en peptides d'avocat va de 0,001% à 5% en poids par rapport au poids total de la composition ; et dans laquelle l'avocat est notamment choisi parmi les variétés *Hass, Fuerte, Ettinger, Bacon, Nabal, Anaheim, Lula, Reed, Zutano, Queen, Criola Selva, Mexicana Canta, Region Dschang, Hall, Booth, Peterson,* et *Collinson Red.*

4. Composition selon l'une quelconque des revendications précédentes, dans laquelle la teneur en extrait d'hamamélis va de 1.10⁻⁴% à 50% en poids par rapport au poids total de la composition ; et dans laquelle l'hamamélis est notamment de l'*Hamamelis virginiana.*

5. Composition selon l'une quelconque des revendications précédentes, dans laquelle l'extrait de polyphénols de graines de passiflore comprend :
- au moins 30% en poids de polyphénols, exprimés en équivalent d'acide gallique, par rapport au poids total de l'extrait sec de polyphénols de graines de passiflore, de préférence encore au moins 35%, de préférence encore au moins 40% en poids de polyphénols, exprimés en équivalent d'acide gallique, par rapport au poids total de l'extrait sec de polyphénols de graines de passiflore ; et
- au moins 10% en poids d'acides organiques, par rapport au poids de l'extrait sec ;
de préférence dans laquelle au moins 50% en poids desdits polyphénols sont des dérivés catéchiques, exprimés en équivalent d'acide gallique, par rapport au poids de polyphénols dans l'extrait sec ;
en particulier dans laquelle lesdits acides organiques sont l'acide acétique, l'acide malique, l'acide citrique ou leurs mélanges.

6. Composition selon l'une quelconque des revendications précédentes, dans laquelle l'extrait de peptides d'avocat présente la composition suivante en acides aminés, en pourcentage de poids par rapport au poids total de l'extrait de peptides d'avocat :
| | |
|---|---|
| - Alanine | 6,4 - 7,8 |
| - Arginine | 4,7 - 5,7 |
| - Acide aspartique | 10,3 - 12,7 |
| - Cystine-cystéine | 2,9 - 3,5 |
| - Acide glutamique | 13,0 - 15,8 |
| - Glycine | 5,3 - 6,5 |
| - Histidine | 2,2 - 2,6 |
| - Isoleucine | 4,8 - 5,8 |
| - Leucine | 7,6 - 9,4 |
| - Lysine | 3,0 - 3,8 |
| - Méthionine | 1,2 - 1,6 |
| - Phénylalanine | 4,7 - 5,7 |
| - Proline | 4,1 - 5,2 |
| - Sérine | 5,5 - 6,7 |
| - Thréonine | 4,6 - 5,6 |
| - Tyrosine | 3,6 - 4,4 |
| - Valine | 5,8 - 7,2. |

7. Composition selon l'une quelconque des revendications précédentes, dans laquelle l'extrait de polyphénols de graines de passiflore est obtenu par extraction solide/liquide des graines de passiflore dans un solvant choisi parmi l'eau, les glycérols, les glycols et leurs mélanges.

8. Composition selon l'une quelconque des revendications précédentes, dans laquelle l'extrait de peptides d'avocat est obtenu par extraction aqueuse de fruits de l'avocatier, et délipidation.

9. Composition selon l'une quelconque des revendications précédentes, dans laquelle l'extrait d'hamamélis est obtenu par extraction aqueuse de feuilles d'hamamélis.

10. Composition selon l'une quelconque des revendications précédentes, comprenant en outre au moins un ingrédient choisi dans le groupe constitué par : le beurre de Karité, l'huile d'avocat, le glycérol, l'alpha-tocophérol et leurs mélanges.

11. Composition selon l'une quelconque des revendications précédentes, **caractérisée en ce qu'**elle se présente sous une forme propre à une administration par voie topique ou par voie orale.

12. Composition selon l'une quelconque des revendications précédentes, comprenant en outre au moins un excipient cosmétiquement acceptable.

13. Utilisation cosmétique d'une composition cosmétique selon l'une quelconque des revendications 1 à 12, pour prévenir l'apparition des vergetures sur la peau et/ou réduire les vergetures sur la peau, en particulier pendant l'adolescence et/ou pendant la grossesse.

14. Méthode de soin cosmétique et/ou esthétique de la peau, pour prévenir l'apparition des vergetures sur la peau et/ou réduire les vergetures sur la peau, en particulier pendant l'adolescence et/ou pendant la grossesse, comprenant l'administration d'une composition cosmétique selon l'une quelconque des revendications 1 à 12.

15. Composition pharmaceutique selon l'une quelconque des revendications 1 à 12, pour son utilisation dans la prévention ou le traitement des vergetures de la peau, la composition comprenant de préférence en outre au moins un excipient pharmaceutiquement acceptable.
